# EUROPEAN PATENT SPECIFICATION

(11) **EP 3 983 429 B1**
(45) Date of publication and mention of the grant of the patent: **15.04.2026**
(21) Application number: 20751675.8
(22) Date of filing: 04.06.2020
(51) Int. Cl.: C07K 14/005, A61K 39/12

(54) **AMINO ACID SEQUENCES OF RECOMBINANT FLAVIVIRAL PROTEINS AND RECOMBINANT FLAVIVIRAL VIRUS-LIKE PARTICLES, THEIR MEDICAL USE IN THE PREVENTION OF ZIKA VIRUS INFECTION AND FOR IN VITRO DIAGNOSIS**
AMINOSÄURESEQUENZEN VON REKOMBINANTEN FLAVIVIRALEN PROTEINEN UND REKOMBINANTEN FLAVIVIRALEN VIRUSÄHNLICHEN PARTIKELN, IHRE MEDIZINISCHE VERWENDUNG ZUR PRÄVENTION VON ZIKA-VIRUS-INFEKTIONEN UND ZUR IN-VITRO-DIAGNOSE
SÉQUENCES D'ACIDES AMINÉS DE PROTÉINES DE FLAVIVIRUS RECOMBINANT ET PARTICULES PSEUDOVIRALES DE FLAVIVIRUS RECOMBINANT, LEUR UTILISATION MÉDICALE DANS LA PRÉVENTION D'UNE INFECTION PAR LE VIRUS ZIKA ET POUR LE DIAGNOSTIC IN VITRO

(30) Priority: 13.06.2019 PL 43023419
(43) Date of publication of application: 20.04.2022
(73) Proprietor: Uniwersytet Gdanski, 80-309 Gdansk (PL)
(72) Inventor: KROL, Ewelina, 81-153 Gdynia (PL); SZEWCZYK, Boguslaw, 80-1156 Gdansk (PL); BRZUSKA, Gabriela, 80-271 Gdansk (PL); CZARNOTA, Anna, 81-017 Gdynia (PL)
(74) Representative: Czarnik, Maciej
(86) International application number: PCT/PL2020/050045
(87) International publication number: WO 2020/251378

(56) References cited:
- WO-A1-2017/150683
- WO-A1-2018/009603
- WO-A1-2018/152526
- PETER NAMBALA ET AL: "Role of Zika Virus prM Protein in Viral Pathogenicity and Use in Vaccine Development", FRONTIERS IN MICROBIOLOGY, vol. 9, 2 August 2018 (2018-08-02), XP055735574, DOI: 10.3389/fmicb.2018.01797
- ANNA DURBIN: "Vaccine Development for Zika Virus-Timelines and Strategies", SEMINARS IN REPRODUCTIVE MEDICINE, vol. 34, no. 05, 8 September 2016 (2016-09-08), US, pages 299 - 304, XP055330014, ISSN: 1526-8004, DOI: 10.1055/s-0036-1592070

## Description

The invention is related to the field of vaccines based on immunogenic antigens used to prevent Zika virus (ZIKV) infection. The invention defines the amino acid sequences of individual recombinant Zika virus proteins or recombinant virus-like particles (VLPs) containing at least two fragments of Zika virus proteins. All sequences are based on common elements. In both types of sequences, the full E structural protein or only the cytoplasmic domain of E protein and / or the full prM structural protein were used. Moreover, in some sequences the fragment of E protein of tick-borne encephalitis virus (TBEV) was used. The invention in the form of amino acid sequences of recombinant proteins or recombinant virus-like particles has a potential therapeutic use - medical use - to prevent Zika virus infection as an immunogenic component for the preparation of vaccines. The invention in the form of recombinant proteins or recombinant virus-like particles with amino acid sequences according to the invention can be also used as *in vitro* diagnostic antigen for the detection of flavivirus infections, in particular Zika virus, tick-borne encephalitis virus and Dengue virus as a component of diagnostic tests. In addition, the invention defines a signal sequence that can be used to overproduce various flaviviral proteins, including recombinant proteins or virus-like particles, the sequences of which are the subject of this invention. The invention further concerns an immunogenic vaccine against Zika virus based on single proteins and / or based on VLPs with amino acid sequences according to the invention.

The genus *Flavivirus* of the family *Flaviviridae* include many human as well as animal pathogens. Most of them are transmitted by means of insect vectors such as ticks e.g. tick-borne encephalitis virus and mosquitoes e.g. Zika virus or Dengue virus.

Zika virus is a pathogen that causes an infectious tropical disease called zika fever. According to the World Health Organization, until 2017 the presence of Zika virus has been confirmed in 87 countries around the world. Zika virus is spread mostly by the bite of an infected *Aedes species* mosquito (*Ae. aegypti* and *Ae. albopictus*), however it can also be transmitted through contact with the blood of an infected person, sexually and from mother to fetus. The incubation period (the time from exposure to symptoms) of Zika virus disease is estimated to be 3-14 days. Most people infected with Zika virus do not develop symptoms. WHO estimates that symptoms of zika fever appear in one of five infected people. Symptoms are generally mild including fever, rash, conjunctivitis, muscle and joint pain, malaise, and headache, and usually last for 2-7 days (Cerbino-Neto i wsp., (2016), Emerg. Infect. Dis. 22, 1318-1320).

A common feature of flaviviruses in infected people are neurological complications. During Zika virus epidemics in 2014-2016, an association between Zika virus infection and microcephaly was reported. Zika virus infection triggers also Guillain-Barré syndrome, neuropathy and myelitis, particularly in adults and older children (Lowe i wsp., (2018), Int. J. Environ. Res. Public. Health 15). The occurrence of such serious complications emphasizes the importance of the through research for the development of a preventive vaccine.

Members of the *Flavivirus* genus share several morphological similarities. Flaviviruses are characterized by a high structural similarity of virions, similarities in the course of the replication cycle, as well as high similarity of viral proteins. Zika virus, like other flaviviruses, is an enveloped virus with a mean diameter of 40-60 nm and is composed of two proteins anchored on the outer side of the virion: membrane protein M and envelope protein E. The mature virions are composed of 90 dimers of E and M protein. The bilayer lipid envelope surrounds the capsid constructed from protein C, in which the single-stranded positive RNA genome [ssRNA(+)] is enclosed. Both prM/M and E proteins are transmembrane proteins with two transmembrane domains at the C' end. E protein consists of an ectodomain, which is divided into three domains (DI, DII, DIII) along with stem and anchor regions. Stem and anchor regions, which comprise almost 20% of the whole E protein, constitute helix and transmembrane domains regions, respectively. DI domain forms β-barrel structure, DII has a finger-like structure, while DIII structurally resembles the structure of an immunoglobulin. Most of flavivirus strains possess one N-glycosylation site in DI. The functional studies on the structure of E glycoprotein of tick-borne encephalitis virus have shown the role of DII in the dimerization process. It has been shown that the stem region is involved in the process of trimerization and the stabilization of prM-E heterodimers in immature particles, while the anchor region is involved in the formation of progeny particles. DIII is involved in receptor binding during entry of the virus into host cells and contains epitopes recognized by neutralizing antibodies and cytotoxic lymphocytes, which makes E-glycoprotein a suitable candidate for use as a vaccine or diagnostic antigen.

During the late steps of the replication cycle in a host cell, the assembly of virions occurs in the rough endoplasmic reticulum, where the cleavage of C protein from prM protein via the viral protease NS2B/NS3 complex is performed. This process is required for subsequent cleavage of prM and E protein by host cell signal peptidase, resulting in the assembly of immature particles composed of heterodimers of prM-E proteins that are organized into 60 trimers, which gives the particle a spiky structure. In the next step, virions are transported to the trans-Golgi network and subsequently packed into vesicles for secretion. The pr fragment of prM protein in immature state of virions covers the fusion loop of E protein, protecting it from premature fusion with membranes during egression through the acidic environment of the trans-Golgi network. Immediately before the release of virions from the host cell, the pr fragment is cleaved by cellular furin protease causing the secretion of mature virus particles. Cleavage of the pr fragment changes the conformation of E protein, forming mature particles (Heinz i Stiasny (2017), Microbiol. Mol. Biol. Rev. 81 e00055-16). Differences in the expression levels of these proteases in various cells that are permissive to flaviviruses give rise to a heterogeneous population of particles: mature and immature virus particles and particles that can be defined as partially mature. It is believed that the presence of immature flavivirus virions in the human body may play a role in the evasion of the immune response. Additionally, during the flavivirus replication cycle, empty particles built from structural proteins, which lack the nucleocapsid and genetic material, are secreted and, similarly to virions, traffic along the same secretory route. The natural assembly process of these particles as well as flaviviral virions was used to produce recombinant virus-like particles in different expression systems. The expression of two structural proteins prM and E leads to the formation of virus-like particles (VLPs). VLPs consist of multiples copies of prM/M and E proteins anchored in the lipid bilayer. The high structural similarity to native virions makes VLPs an excellent candidate for vaccine production in contrast to live attenuated or inactivated viruses. Lack of genetic material makes VLPs non-infectious, and at the same time they are highly immunogenic, as they contain many copies of viral structural proteins, strictly ordered, which ensures the appropriate presentation of epitopes being the target of neutralizing antibodies or cytotoxic lymphocytes.

Since the last epidemic, special efforts have been made to develop a potential vaccine against Zika virus. Currently, different types of vaccine antigens are under investigation, includ- ing DNA vaccines, mRNA vaccines, viral vectors encoding ZIKV genes, and inactivated or live-attenuated viruses. All tested antigens are based on ZIKV structural proteins, usually E-glycoprotein. Larocca et al. (2016) described the production of a DNA vaccine based on a plasmid vector encoding the M and E proteins genes of Zika virus (Larocca i wsp., (2016), Nature 536, 474-478). The immunogenicity of various variants of the M and E protein genes was studied, including the deletions in the stem and anchor region, in the mouse model. The plasmid containing the full sequence of ZIKV structural proteins has been described as the most immunogenic. In addition, the immunogenicity of M and E proteins was compared to various viral vectors, i.e. plasmid or adenoviral vector and inactivated ZIKV particles in non-human primates. All antigens induced high levels of neutralizing antibodies which were sufficient to provide animals protection, however, the antigen based on the adenoviral vector elicited the strongest immune response (Abbink i wsp. (2016), Science 353, 1129-1132). Dowd et al. (2016) described the production of DNA vaccine based on a plasmid vector carrying the full sequence of Zika virus prM and E genes (VRC 5283), as well as with modifications in the signal sequence and the transmembrane domains of E protein (VRC 5288) (Dowd i wsp. (2016), Science 354, 237-240 ). The nucleotide sequences of these regions have been exchanged to the sequences corresponding to the same regions derived from the Japanese encephalitis virus. The immunogenicity of these antigens was tested *in vivo* in a mouse model and in non-human primates, followed by clinical trial. In Phase I clinical trials, VRC 5283 vaccine have been tolerated better by patients, and therefore in 2017 the Phase II of clinical trials had been started (Gaudinski i wsp. (2018), The Lancet 391, 552-562). Further studies on DNA anti-Zika vaccine were also based on prM and E genes, however with mutations in the N-glycosylation region and with the replacement of the signal sequence with the IgE sequence (GLS 5700) (Muthumani i wsp. (2016), Npj Vaccines 1, 16021). This antigen was also tested *in vivo* in a mouse model and in non-human primates, and further it was passed to Phase I clinical trials (Tebas i wsp. (2017), N. Engl. J. Med. 10, 1). Kim et al. (2016) described the adenoviruses encoding the extracellular domain of E protein as a viral vector vaccine and as a vector to produce recombinant protein in mammalian cells (Kim i wsp. (2016), EBioMedicine 13, 315-320).

In recent years, several attempts to construct vaccine candidates based on recombinant ZIKV structural proteins or virus-like particles have been reported. Zika VLPs were constructed by coexpression of prM-E or C-prM-E proteins together with a complex of NS2B/NS3 virus protease in mammalian cells. Their immunogenicity was further investigated in a mouse model also in comparison with a DNA vaccine carrying the same genes, as described in Boigard et al. (2017), PLoS Negl. Trop. Dis. 11, e0005608 oraz Garg et al. (2017), J. Virol. JVI.00834-17. Immunogenicity studies of truncated E protein variants overproduced in a subunit form or fused with hepatitis B virus-like particles or IgG immunoglobulin Fc fragment have also been performed (Han i wsp. (2017), Sci. Rep. 7, 10047; Tai i wsp. (2018), Microbes Infect. 7, 7; Yang, i wsp. (2017), Sci. Rep.7). The prM-E VLPs were also obtained in insect cells (Sf9). Although, these particles were immunogenic (induction of cellular and hu- moral response), the level of protection was not as high as for inactivated virus (Dai i wsp. (2018), Virologica Sinica 33, (3)). Sf9 cells were also employed for the construction of VLPs consisting of only ZIKV E protein (Dai i wsp. (2018), Viruses, 10(7)).

Other studies have also been undertaken to produce mRNA-based vaccines carrying Zika virus prM-E protein genes. Antigenic and immunogenic properties of different prM-E gene variants with modifications in the fusion loop region and the effect of encapsulation of mRNA particles into liposomes were further investigated in comparison with live attenuated ZIKV (Richner i wsp. (2017a) Cell 168, 1114-1125.e10; Richner i wsp. (2017b), Cell 170, 273-283.e12). The mRNA-based vaccine encoding the prME genes is currently in Phase II clinical trials.

Another *in vivo* mouse study analysed the immunogenicity of a vaccine based on a live attenuated Japanese chimeric encephalitis virus, where the genes of the prM-E structural proteins were replaced by genes of Zika virus (Li i wsp. (2018), Nat. Commun. 9, 673). Similar tests have also been carried out for other flaviviruses and in all cases these viruses have elicited a strong immune response (Li i wsp. (2013a), J. Virol. 87, 13694-13705; Li i wsp. (2013b), J. Gen. Virol. 94, 2700-2709; Wang i wsp. (2014), Vaccine 32, 949-956). In addition, Phase I clinical trials are being in progress with inactivated ZIKV (Modjarrad i wsp. (2018), The Lancet 391, 563-571).

Many approaches have been applied to produce recombinant proteins and VLPs of flaviviruses, which can be further used as vaccine or diagnostic antigens. These strategies are based on the expression of C, prM and/or E protein in natural or modified - recombinant form.

The invention refers to the new strategy for the production of immunogenic particles i.e. recombinant flaviviral proteins and recombinant virus-like particles. These strategies include the combination of natural amino acid sequences of Zika virus prM and/or E proteins with other additional elements, i.e. an optimized signal sequence and/or the natural P2A peptide sequence together with the GSG linker being the glycine-serine-glycine amino acid combination and/or the sequence of transmembrane domain of E protein of tick-borne encephalitis virus. The introduced modifications improve the production, assembly, secretion, maturation and immunogenicity of recombinant proteins and VLPs. Recombinant proteins as well as VLPs based on the amino acid sequences presented in this invention can be overproduced in different expression systems in mammalian, insect, yeast, or protozoal cells.

### The invention is disclosed in the claims.

The first aspect of the present invention relates to the two amino acid sequences of the recombinant flaviviral VLPs shown as the sequence number SEQ ID NO 9 and 10 consisting of two naturally occurring Zika virus structural protein - prM and E or its fragment - the cytoplasmic domain where at least two modifications into the amino acid sequence has been introduced by adding an additional fragment into the construct. The invention presents the individual amino acid sequences of recombinant VLPs (SEQ ID NO: 9 and 10), wherein the change in the natural protein sequence refers to the introduction of additional modified sequences: the optss signal sequence (SEQ ID NO: 11), P2A sequence and additionally in SEQ ID NO: 10 TBEV stem/anchor domain. These modifications result in more efficient overproduction of VLPs and increase their immunogenicity.

A second aspect of the invention relates to the recombinant VLPs with the ammo acid sequences according to the invention (SEQ ID NO: 9 and 10) for medical use - for use in the prevention of Zika virus infections, especially when used in a form of a vaccine component. This aspect includes the composition of immunogenic preparation comprising at least one flaviviral antigen VLPs, and preferably an adjuvant.

The present invention also includes the antig enic compositions for the prevention of flavivirus infections comprising at least one recombinant VLPs with the amino acid sequence SEQ ID NO: 9 and/or SEQ ID NO 10.

Amino acid sequences of the recombinant VLPs (SEQ ID NO: 9 and 10) according to the invention are the amino acid sequences of the structural proteins of the Flavivirus viruses, to which various sequence modifications have been introduced.

The structural prM and E proteins of Zika virus are the natural components of virions. These proteins can also form VLPs, which consist of at least two structural proteins. In Zika virus genome, the prM and E protein genes follow the structural C protein gene. The transmembrane domain of the C protein is the natural signal sequence for the prM (ss1) protein, and similarly the transmembrane domain of the prM protein is the natural signal sequence for the E (ss) protein. These signal sequences direct proteins to appropriate compartments in a eukaryotic cell for further maturation, assembly into viral particles, and secretion outside the cell. The signal sequences, after fulfilling their role, are cut off by signal peptidases appropriate for a cell type. Therefore, in order to improve the properties of recombinant proteins, the artificial signal sequence optss (SEQ ID NO: 11) for recombinant prM or E proteins or VLPs (SEQ ID NO: 9, 10) was used to enhance the efficient overproduction and efficient maturation and secretion of recombinant VLPs in various eukaryotic cells. In the host cell after translation, the amino acid sequence of optss (SEQ ID NO: 11) is efficiently cleaved by the signal peptidases from immature recombinant prM or E proteins resulting in increased secretion and further maturation of proteins or VLPs (SEQ ID NO: 9 and 10).

Another modification is the introduction of a natural peptide sequence (P2A) responsible for the self-acting separation of the eo-maturing recombinant prM and E proteins,
which can form VLPs, eliminating the dependence of this process on the presence of the appropriate signal peptidase in the eukaryotic cell (SEQ ID NO: 9 and 10). Effective separation of prM and E proteins is essential for the full maturation of virus-like particles. The P2A peptide sequence originates from the porcine teschovirus-1. It is a 19-amino acid peptide terminated with proline-glycine-proline amino acids. The presence of these amino acids in the protein cassette causes the termination of translation at the first proline site and restart of translation from the next proline, resulting in the production of two separate recombinant proteins. In addition, the "GSG linker" between a prM protein and P2A peptide was introduced. This linker is a sequence of three amino acids glycine-serine-glycine, between the C'-end of the prM protein and the N'-end of the P2A peptide to reduce spherical disorders of the protein structure.

The invention further comprises the amino acid sequences of VLPs, where the natural amino acid sequence of the cytoplasmic domain of E protein of Zika virus has been combined with the natural amino acid sequence of the helical and transmembrane domains of tick-borne encephalitis virus E protein (TBEV stem/anchor) (SEQ ID NO: 10). This combination allows for more efficient assembly of recombinant virus-like particles and increases the immunogenicity of recombinant proteins and VLPs.

The combination of all introduced elements optss (SEQ ID NO: 11), P2A and TBEV stem/anchor) to the sequence of prM and E protein of Zika virus is innovative and has never been used to produce such VLPs. Moreover, such VLPs have never been proposed as immunogens for prophylaxis of Zika virus.

Based on the natural ability of prM and E proteins to form VLPs, on the previous research with flaviviral VLPs as well as on our own experience in the production of viral particles performed at the Department of Recombinant Vaccines at the Intercollegiate Faculty of Biotechnology University of Gdansk and Medical University of Gdansk, a new and effective strategy for the construction of flaviviral recombinant proteins or VLPs containing at least one of the structural proteins of flaviviruses, especially Zika virus using a combination of the above-mentioned elements was designed.

The combinations of the presented modifications m the ammo acid sequences of flaviviral proteins, i.e. the amino acid sequences of the invention, are shown in the Scheme 1. The amino acid sequences of prM and E proteins of ZIKV can be derived from different strains of Zika virus. The amino acid sequences of the Zika virus strain BeH818995 (GenBank: AMA12084.1) were used in the recombinant proteins and VLPs presented in the invention. 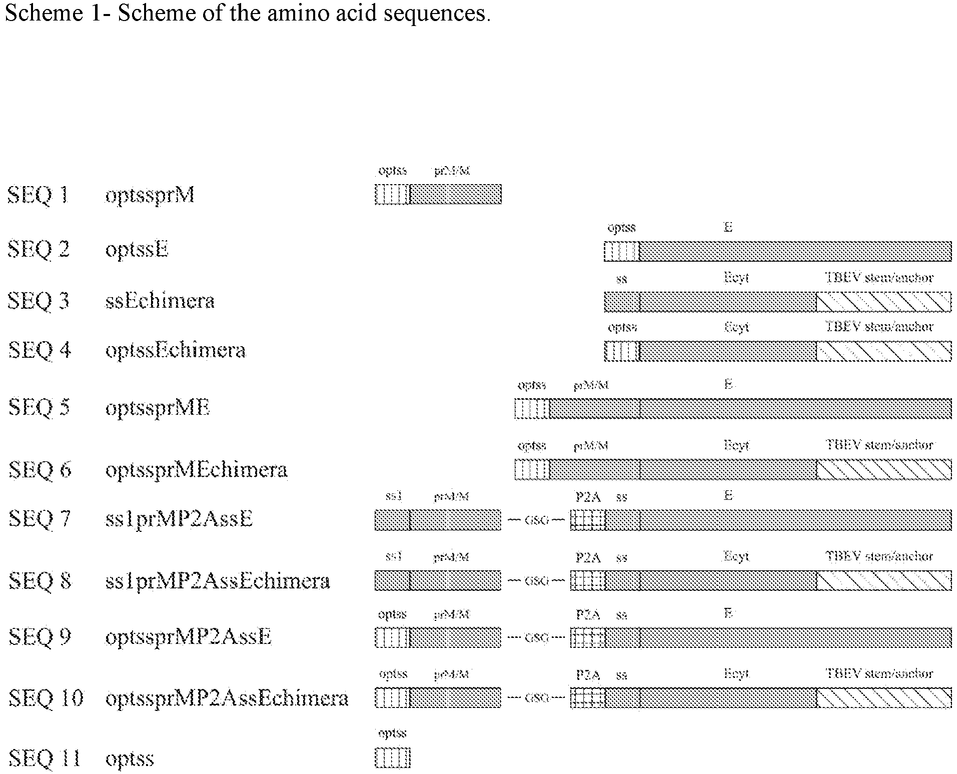
optss - signal sequence optimized on the basis of the signal sequence present in transmembrane domains of Zika virus protein C
ss1 - signal sequence for prM/M protein present in transmembrane domains of Zika virus protein C
prM - structural protein of Zika virus
GSG - linker sequence of three amino acids resulting in more effective separation of proteins
P2A - sequence resulting in self-acting separation of protein, it comes from swine Teschovirus-1
ss - signal sequence for Zika virus protein E present in transmembrane domains of Zika virus prM protein
Ecyt - cytoplasmic domain of Zika virus protein E
E- structural protein of Zika virus
TBEV stem/anchor - helical and transmembrane domains of E protein of tick-borne encephalitis virus (TBEV)

### Number of sequences:

### SEQ 1

The amino acid sequence of the recombinant prM protein of Zika virus comprising the amino acid sequence of the natural Zika virus prM protein (region 123-291 of the amino acid sequence of the Zika virus polyprotein) with the optimized amino acid signal sequence optss (SEQ 11) attached.

### SEQ 2

The amino acid sequence of the recombinant E protein of Zika virus comprising the amino acid sequence of the natural Zika virus E protein (region 292-794 of the amino acid sequence of the Zika virus polyprotein) with the optimized amino acid signal sequence optss (SEQ 11) attached.

### SEQ 3

The amino acid sequence of the recombinant chimeric flaviviral protein - the amino acid sequence consisting of the natural amino acid signal sequence ss (region 105-122 of the amino acid sequence of the Zika virus polyprotein), amino acid sequence of the cytoplasmic domain of the natural Zika virus E protein (Ecyt) (region 292-697 of the amino acid sequence of the Zika virus polyprotein) fused to the amino acid sequence of the helical and transmembrane domains of E protein of tick-borne encephalitis virus (TBEV) (TBEV stem/anchor) (region 680-776 of the amino acid sequence of TBEV polyprotein, GenBank: AAA86870.1)

### SEQ 4

The amino acid sequence of the recombinant chimeric flaviviral protein - the amino acid sequence consisting of the optimized amino acid signal sequence optss (SEQ 11), amino acid sequence of the cytoplasmic domain of the natural Zika virus E protein (Ecyt) (region 292-697 of the amino acid sequence of the Zika virus polyprotein) fused to the amino acid sequence of the helical and transmembrane domains of E protein of tick-borne encephalitis virus (TBEV) (TBEV stem/anchor) (region 680-776 of the amino acid sequence of TBEV polyprotein, GenBank: AAA86870.1)

### SEQ 5

The amino acid sequence of recombinant VLPs - the amino acid sequence consisting of the amino acid sequence of two proteins: Zika virus prM and E (region 123-794 of the amino acid sequence of the Zika virus polyprotein) preceded by the optimized amino acid signal sequence optss (SEQ 11)

### SEQ 6

The amino acid sequence of recombinant VLPs - the amino acid sequence consisting of the amino acid sequence of two proteins: prM protein of Zika virus (region 123-291 of the amino acid sequence of the Zika virus polyprotein) and chimeric flaviviral protein consisting of the cytoplasmic domain of the natural Zika virus E protein (Ecyt) (region 292-697 of the amino acid sequence of the Zika virus polyprotein) fused to the helical and transmembrane domains of E protein of tick-borne encephalitis virus (TBEV) (TBEV stem/anchor) (region 680-776 of the amino acid sequence of TBEV polyprotein, GenBank: AAA86870.1) preceded by the optimized amino acid signal sequence optss (SEQ 11)

### SEQ 7

The amino acid sequence of recombinant VLPs - the amino acid sequence consisting of the amino acid sequence of two proteins: prM protein of Zika virus (region 123-291 of the amino acid sequence of the Zika virus polyprotein) and E protein of Zika virus (region 292-794 of the amino acid sequence of the Zika virus polyprotein) preceded by the natural amino acid signal sequences ss1 (region 105-122 of the amino acid sequence of the Zika virus polyprotein) and ss (region 273-291 in the amino acid sequence of the Zika virus polyprotein), separated by a GSG linker sequence linked to the P2A peptide (region 979-997 of the amino acid sequence of the Teschovirus 1 virus polyprotein, Genbank: NP_653143.1)

### SEQ 8

The amino acid sequence of recombinant VLPs - the amino acid sequence consisting of the amino acid sequence of two proteins: prM protein of Zika virus (region 123-291 of the amino acid sequence of the Zika virus polyprotein) and chimeric flaviviral protein consisting of the cytoplasmic domain of the natural Zika virus E protein (Ecyt) (region 292-697 of the amino acid sequence of the Zika virus polyprotein) fused to the helical and transmembrane domains of E protein of tick-borne encephalitis virus (TBEV) (TBEV stem/anchor) (region 680-776 of the amino acid sequence of TBEV polyprotein, GenBank: AAA86870.1) preceded by the natural amino acid signal sequences ss1 and ss (region 105-122 and 273-291of the amino acid sequence of the Zika virus polyprotein, respectively) separated by a GSG linker sequence linked to the P2A peptide (region 979-997 of the amino acid sequence of the Teschovirus 1 virus polyprotein, Genbank: NP_653143.1)

### SEQ 9

The amino acid sequence of recombinant VLPs - the amino acid sequence consisting of the amino acid sequence of two proteins: prM protein of Zika virus (region 123-291 of the amino acid sequence of the Zika virus polyprotein) and E protein of Zika virus (region 292-794 of the amino acid sequence of the Zika virus polyprotein) preceded by the optimized amino acid signal sequence optss (SEQ 11) and the natural amino acid signal sequences ss (region 273-291 in the amino acid sequence of the Zika virus polyprotein), separated by a GSG linker sequence linked to the P2A peptide (region 979-997 of the amino acid sequence of the Teschovirus 1 virus polyprotein, Genbank: NP_653143.1)

### SEQ 10

The amino acid sequence of recombinant VLPs - the amino acid sequence consisting of the amino acid sequence of two proteins: prM protein of Zika virus (region 123-291 of the amino acid sequence of the Zika virus polyprotein) and the recombinant chimeric flaviviral protein consisting of the cytoplasmic domain of the natural Zika virus E protein (Ecyt) (region 292-697 of the amino acid sequence of the Zika virus polyprotein) fused to the helical and transmembrane domains of E protein of tick-borne encephalitis virus (TBEV) (TBEV stem/anchor) (region 680-776 of the amino acid sequence of TBEV polyprotein, GenBank: AAA86870.1) preceded by the optimized amino acid signal sequence optss (SEQ 11) and the natural amino acid signal sequences ss (region 273-291 in the amino acid sequence of the Zika virus polyprotein), separated by a GSG linker sequence linked to the P2A peptide (region 979-997 of the amino acid sequence of the Teschovirus 1 virus polyprotein, Genbank: NP_653143.1)

### SEQ 11

The optimized amino acid signal sequence directing flaviviral proteins for efficient secretion (optss)

The signal sequence optss (SEQ 11) is an optimized 16 amino acid sequence directing flaviviral proteins for efficient secretion, which was developed based on the amino acid sequence of the transmembrane domains of the capsid protein C - region 105-122 in the amino acid sequence of the Zika virus polyprotein (ss1). The sequence optimization was carried out by increasing the amount of hydrophobic amino acids such as alanine, valine, glycine and leucine in the ss1 sequence (Scheme 2) and shortening the ss1 sequence. The similarity of both signal sequences is less than 40%.

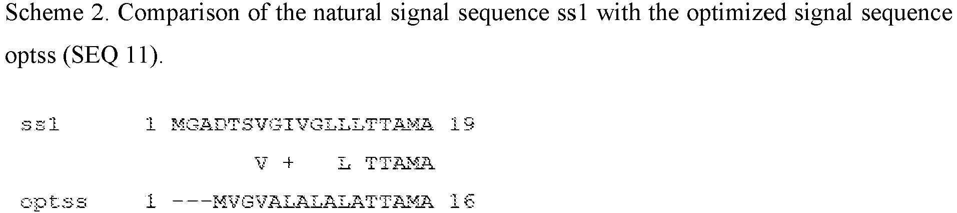

In summary, the introduced modifications to the amino acid sequences of the known flaviviral proteins described in this invention offers the following advantages:
- the optss signal sequence facilitates the secretion of mature prM or E proteins (SEQ 1, 2, 4) or virus-like particles VLPs (SEQ 5, 6, 9, 10), which allows for the usage of the simpler methods of protein/VLPs purification
- the P2A peptide inserted between prM and E proteins (SEQ 7- 10) allows for more efficient separation of flaviviral prM and E proteins
- the optss signal sequence attached to the prM protein and P2A peptide inserted between the prM and E proteins (SEQ 9, 10) increases the efficiency of recombinant flaviviral VLPs production in various eukaryotic expression systems (in insect, mammalian, protozoal and yeast cells)
- the TBEV stem/anchor region increases the immunogenicity of antigens (SEQ 3, 4, 6, 8, 10)
- the presence of the TBEV stem/anchor region in the E protein of Zika virus and the presence of P2A sequence between prM and E proteins (SEQ 8, 10) improves the efficiency of virus-like particles assembly, stabilizes the virus-like particles and allows for the change of their antigenic properties

Virus-like particles (VLPs) based on SEQ 5-10 sequences can elicit a strong immune response in a mouse model, which is necessary for the effective neutralization of Zika virus in both *in vitro* and *in vivo* models, which translates into the neutralization of Zika virus in the human body.

The replacement of the transmembrane domains of Zika virus E protein with the transmembrane domains of E protein of TBEV as well as the use of P2A peptide increases the maturation of recombinant VLPs based on SEQ 7-10 amino acid sequences that can be further used as vaccine antigen, reducing the presence of antibody-dependent enhancement of flaviviral infection (ADE) after immunization or infection with another flavivirus.

VLPs (SEQ ID NO: 9 and 10) according to the invention can be overproduced in insect cells (e.g. Sf9, HiS, Sf21), mammalian cells (e.g. HEK293T, BHK21, CHO), yeast cells (including Pichia pastoris) or Leishmania tarantolae protozoan cells. Commercially available systems, e.g. Bac-to-Bac^{®} (Invitrogen) and LEXSY system (Jena Bioscience) can be used for overproduction of proteins or VLPs in insect cells and protozoal cells, respectively.

For the overproduction of flaviviral antigens, standard conditions (e.g. composition of culture medium, overproduction time, cell culture temperature, amount of vector, etc.) used for overproduction of viral proteins and/or conditions recommended by the manufacturers of commercially available systems may be used.

A second aspect of this invention concerns the use of VLPs based on SEQ ID NO: 9 and 10 according to the invention and the use of their mixtures as vaccine antigens in the prophylaxis of Zika virus infections. This aspect also includes a composition of an immunogenic formulation comprising VLPs and at least one adjuvant - a component of the vaccine.

The potential vaccine for prevention of Zika virus infection may include produced recombinant VLPs based on SEQ ID NO: 9 and/or 10 sequences mixed in a 1:1 ratio with the adjuvant. Recombinant VLPs can be produced according to the production method presented in Example 1.

The schedule of administration of such vaccine may include 1 to 3 doses administered in 2 weeks intervals. One dose may contain 1-100 µg of total protein in the sample suspended in 50-100 µl of PBS buffer mixed in a 1:1 volume ratio with an adjuvant.

Vaccine preparations may be administered intramuscularly.

The invention is shown in the sequences hereinafter referred to as SEQ ID NO: 9-11 . The invention has also been illustrated with the following exemplary, though not exhaustive, embodiments and figures that show:
Fig. 1. Western blot analysis showing the production of recombinant optssprM protein (SEQ 1) (A) and recombinant proteins optssE, ssEchimera and optssEchimera (SEQ 2, 3, 4) (B) in mammalian cell culture medium.
Fig. 2. Western blot analysis showing the production of ss1prMP2AssE (SEQ 7), optssprMP2AssE (SEQ 8) and optssprMP2AssEchimera (SEQ 9) recombinant VLPs in insect cells.
Fig. 3. Transmission electron microscopy of optssprMP2AssE (SEQ 9) and optssprMP2AssEchimera (SEQ 10) VLPs.
Fig. 4. The graph showing mean titers of mouse sera obtained after 3 immunizations with VLPs (SEQ 9 and 10).
Fig. 5. The graph showing mean absorbance values obtained in the diagnostic test.

### Example 1

### PRODUCTION OF RECOMBINANT PROTEINS (1.a) OR VLPs (1.b) WITH AMINO ACID SEQUENCES OF THE INVENTION

### 1.a

Sequences were obtained in a known procedure.

Genes encoding the recombinant optssprM, optssE, ssEchimera and optssEchimera proteins (SEQ 1, 2, 3, 4 sequences, respectively), were chemically synthesized by GeneArt (Life Technologies Inc. USA). Then, the synthesized fragments were cloned into the pcDNA 3.1 (+) plasmid vector - a plasmid vector for the expression of protein genes in mammalian expression system (Life Technology) using BamHI/EcoRI restriction sites. The correctness of the insert introduction was verified by sequencing.

The plasmids were propagated in *E. coli* (TOP10) cells and isolated using a Plasmid Mini kit (A&A Biotechnology). Subsequently, to produce recombinant proteins, HEK293T mammalian cells were transfected using the jetPRIME^{®} transfection reagent (Polyplus-transfection^{®}) according to the manufacturer's protocol. Transfected mammalian cells were cultured in Dulbecco's Modified Eagle Medium (DMEM) (Lonza) supplemented with 10% bovine serum albumin (FBS) (Gibco ^{™}) at 28°C. After 3 days of incubation, the supernatant culture medium containing the recombinant proteins was harvested.

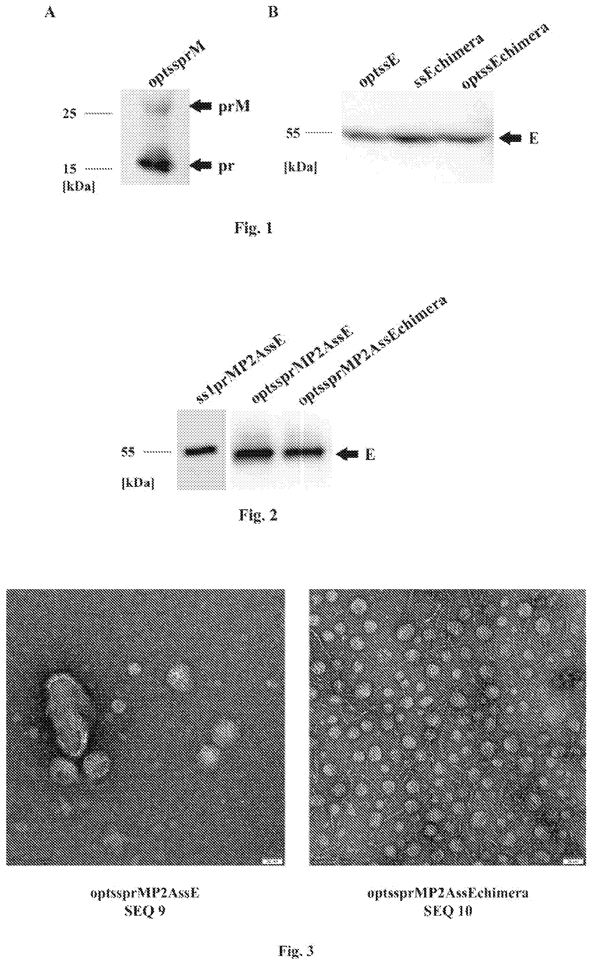

In the next step, the supernatant medium was centrifuged to get rid of cell debris (5000 rpm, 10 min). Further, the polyethylene glycol 6000 to a concentration of 10% and sodium chloride to a concentration of 0.3 M were added to the supernatant and incubated overnight at 4°C. Protein precipitates were then centrifuged for 45 min at 8000 rpm. The precipitates were dissolved in Tris-NaCl-EDTA buffer (50 mM-100 mM-10 mM). Western blot analysis to detect produced protein was performed using specified antibodies directed against this protein. Anti-prM polyclonal serum was used to detect recombinant protein with SEQ 1 sequence (Fig. 1 A) and monoclonal antibodies directed against E protein were used to confirm the presence of recombinant proteins with SEQ 2, 3, 4 sequence in mammalian cell culture medium (Fig. 1 B). The presence of proteins based on SEQ1-4 sequences was confirmed.

In a similar way, a modified signal sequence shown in SEQ11, which is also found in some of the other sequences was confirmed.

### 1.b

Genes coding for recombinant VLPs: optssprME (SEQ 5), optssprMEchimera (SEQ 6), ss1prMP2AssE (SEQ 7), ss1prMP2AssEchimera (SEQ 8), optssprMP2AssE (SEQ 9), optssprMP2AssEchimera (SEQ 10) were chemically synthesized by GeneArt (Life Technologies Inc. USA). Then, the synthesized fragments were cloned into the pFastBac1 plasmid vector - a component of the Bac-to-Bac^{®} expression system (Life Technologies Inc. USA) using BamHI/EcoRI restriction sites.

The plasmid was propagated in *E. coli* (TOP10) cells and isolated using a Plasmid Mini kit (A&A Biotechnology). Then, the recombinant plasmids were used to transform *E. coli* DH10Bac cells containing bacmid DNA to generate recombinant bacmid DNA carrying the genes encoding for VLPs. Bacmid DNA was isolated using the alkaline lysis method. Then, to generate recombinant baculovirus vectors, the transfection of Sf9 insect cell was performed using the Lipofectin Transfection Reagent (Life Technologies Inc. USA) according to the manufacturer's protocol. Sf9 insect cells were cultured in HyClone ^{™} SFX-Insect ^{™} Media (GE Healthcare) at 27°C. After 3 days of incubation, culture medium containing recombinant baculoviruses was harvested. Recombinant baculoviruses were propagated in Sf9 cells for 3-4 passages using a low MOI = 0.1. The baculoviral stock titer was determined by the TCID50 method using Sf9 Easy Titer cells.

For overproduction of VLPs based on SEQ 7, 9 and 10, Sf9 insect cells were infected with recombinant baculoviruses with MOI = 3, culture scale 50 ml, cell density 2x10⁶/ml. The cells were then incubated for 3 days at 27°C.

In a next step, the supernatant medium was centrifuged to get rid of cell debris and the baculovirus vector (5000 rpm, 10 min and 8000 rpm, 90 min). Further, polyethylene glycol 6000 to a concentration of 10% and sodium chloride to a concentration of 0.3 M were added to the supernatant and incubated overnight at 4°C. Protein precipitates were then centrifuged for 45 min at 8000 rpm. The precipitates were dissolved in Tris-NaCl-EDTA buffer (50 mM-100 mM-10 mM). Western blot analysis was performed using anti-E protein antibodies to confirm the presence of recombinant VLPs based on SEQ 7, 9, 10 sequences in the culture medium (Fig. 2). In order to purify the VLPs, molecular filtration was performed using a CL4B resin. VLPs were eluted from the resin using a phosphate buffered saline solution pH 7.4 (PBS) with trisodium citrate at a concentration of 0.32%.

Analysis of the fractions containing VLPs ss1prMP2AssE (SEQ 7), optssprMP2AssE (SEQ 9), optssprMP2AssEchimera (SEQ 10) after molecular filtration was performed using various methods e.g. western blotting, coomassie brilliant blue staining, dynamic light scattering, protein concentration microscopy, target electron microscopy. The results from electron microscopy are shown in Fig. 3.

Similar procedure was followed for the remaining VLPs constructs (SEQ 5, 6 and 8). They were obtained as described above.

The production of VLPs based on SEQ 5, 6 and 8 can be also confirmed by the fact that they contain the same constituent elements (prM, protein E or cytoplasmic domain of E, ss1, optss, P2A and TBEV stem/anchor) in various combinations as it is in VLPs based on SEQ 7, 9 and 10. For this reason, the results shown in the figures using VLPs based on SEQ 7, 9, 10 confirm the possible production of the remaining VLPs due to the fact that VLPs based on SEQ 7, 9 and 10 contain all components of the constructs, while VLPs based on SEQ 5, 6 and 8 contain lower amount of these common components.

All constructs according to the invention can be efficiently obtained by the method presented.

### Example 2

### PREPARATION OF MIXTURES - A COMPOSITION OF AT LEAST ONE RECOMBINANT PROTEIN OF AT LEAST ONE VLPs.

Compositions - mixtures are obtained by mixing at least one recombinant protein of SEQ 1-4 with at least one VLPs of SEQ 5-10.

For example, one of the compositions is obtained by mixing the recombinant protein of SEQ 2 obtained by the method presented in Example 1a in the form of a precipitate dissolved in Tris-NaCl-EDTA buffer with VLPs of SEQ 10 produced by the method presented in Example 1b in the form of precipitate dissolved in Tris-NaCl-EDTA buffer in a 1:1 volume ratio. Similarly, other mixtures of recombinant proteins (SEQ 1, 2, 3, 4 and 5) with recombinant VLPs (SEQ 5, 6, 7, 8, 9 and 10) were obtained.

To confirm the production, the procedure described in the previous example was followed.

### Example 3

### VACCINE PREPARATION

The structure of the sequences according to the invention allows their medical application for the prophylaxis of Zika virus infections from the *Flavivirus* genus, especially as a component of the vaccine.

For example, recombinant optssprMP2AssE (SEQ 9) and optssprMP2AssEchimera (SEQ 10) VLPs produced by the method presented in Example 1b, were used as immunogens in the mouse model studies.

Vaccine preparations were administered to mice in 3 doses with an interval of 2 weeks.

The single dose of vaccine was made by mixing the purified recombinant VLPs (SEQ 9 or SEQ 10) (15 µg total amount of purified protein in 100 µl volume) with AddaVax adjuvant in a 1:1 volume ratio. Two groups of BALB/c mice (6 female mice per group) were inoculated intramuscularly with the antigen/adjuvant mixture at two injection sites. The control group of three BALB/c mice was inoculated with a PBS buffer/adjuvant mixture in the same ratio.

After the first inoculation, the next inoculations (2 and 3) were made by mixing 10 µg of purified recombinant VLPs optssprMP2AssE (SEQ 9) or optssprMP2AssEchimera (SEQ 10) in a volume of 50 µl mixed in a 1:1 volume ratio with AddaVax adjuvant. The preparations were also administered intramuscularly at one injection site.

To determine the efficacy of potential vaccine, i.e. the immunogenicity of recombinant VLPs, all mice were bled and the collected sera after each vaccination (serum 1, 2, 3) of each group of mice were used for immunogenicity analysis by determining the serum titer. The titre was determined by ELISA test using prM/E proteins overproduced in HEK293T mammalian cells and purified by molecular filtration as antigens. Briefly, M-96 plate was coated overnight with 2 µg/ml of antigen at 50 µl. Then, the blocking solution (2% milk in PBS buffer) was added (200 µl). After washing, serial dilutions of sera obtained from individual mice were added in a volume of 50 µl and incubated for 2 h. The plate was washed, and anti-mouse horseradish peroxidase (HRP)-conjugated secondary antibodies were added for 1 h. Antigen-antibody complexes were detected using tetramethylbenzidine substrate (TMB) (Life Technologies Inc USA). The reaction was then stopped by adding 0.5M sulfuric acid. The reaction was read by reading the absorbance at 450 nm in a plate reader.

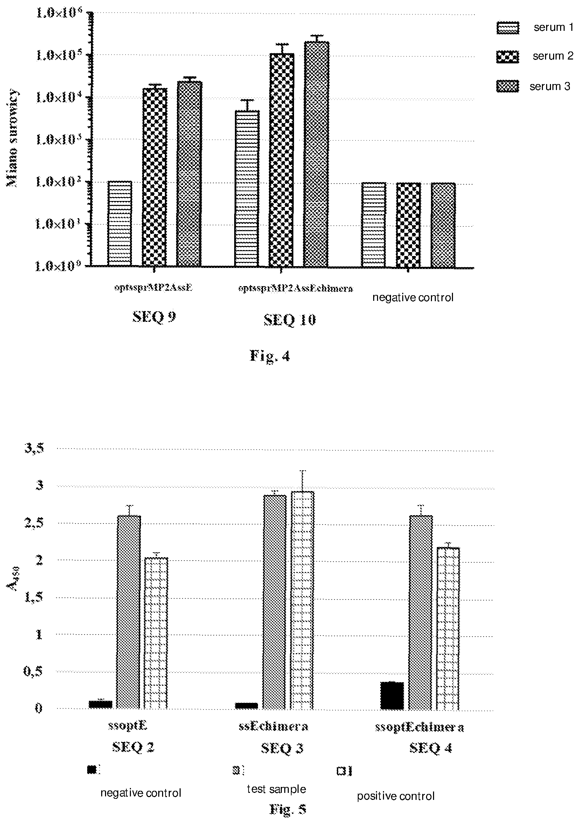

The serum titer was determined by selecting a dilution of serum in which the signal is three times higher than the signal in the appropriate dilution of sera from control mice. The serum titres are presented in Figure 4. After the third vaccination, the average serum titers range from about 10⁴ to over 10⁵. The obtained experiment results confirm the immunogenicity of the obtained VLPs (SEQ 9, 10).

Plaque reduction neutralization test (PRNT test) was also performed to further confirm the usefulness of VLPs based on the sequences of the invention as a potential vaccine - representative sequences, i.e. SEQ 9 and SEQ 10, were selected for the study. Briefly, Vero E6 cells were seeded on the M-24 plate. Next day, 50 pfu of Zika virus (strain MR766) in 100 µl of the DMEM medium was incubated with the same volume of serial dilutions of pooled mouse sera (from last bleeding after immunization with VLPs of SEQ 9 or SEQ 10 sequences) for 2 h in 37°C. Next, the mixtures were incubated with the seeded Vero E6 cells for another 2 h and then exchanged to 1% methylcellulose in MEM medium. Cells were incubated for 5 days in 37°C. The test was done in duplicates. Plaques were visualised with the amid black and counted. Medium values were used for calculation of 90% neutralization value (EC 90) using GraphPad Prism software (Table 1).

**Table 1. Neutralization of Zika virus using mouse sera obtained after vaccination with ssoptprMP2AssE - SEQ 9 and optssprMP2AssEchimera - SEQ 10.**

| Group | EC 90 |
|---|---|
| ssoptprMP2AssE SEQ 9 | 1: 23 |
| optssprMP2AssEchimera SEQ 10 | 1: 32 |
| Negative control | 1: 20 |

Similar laboratory tests have also confirmed that the remaining VLPs (SEQ 5-8) show similar immunogenicity as VLPs based on SEQ 9, 10 sequences due to the presence of the highly immunogenic cytoplasmic domain of E protein, exchange of the transmembrane domains and due to the efficient particle production using the optimized signal sequence and the use of P2A peptide, which results in the efficient separation of prM and E proteins.

Similar laboratory studies have also shown that the use of recombinant proteins (SEQ 2-4) in eliciting the immune response provides a high level of antibodies directed against these proteins after immunization due to the presence of the highly immunogenic cytoplasmic domain of E protein, exchange of transmembrane domains and thanks to the use of the optimized signal sequence for the overproduction of proteins.

The potential application of the vaccine combination for the purposes of prevention/prophylaxis by mixing recombinant proteins (SEQ 1-4) and VLPs (SEQ 5-10) will provide better protection against the virus due to a stronger stimulation of the immune response, which is the result of the presence of the E protein cytoplasmic domain in both recombinant proteins and VLPs. Both the recombinant protein and VLPs present in the potential vaccine can be produced and purified according to the method described in Example 1. Mixtures can be obtained as shown in Example 2.

### Example 4

### DIAGNOSTIC TEST

The structure of the sequences according to the invention indicates their application as a diagnostic antigen for the diagnosis of infections caused by the viruses from the *Flavivirus* genus.

The example demonstrates the utility of recombinant optssE (SEQ 2), ssEchimera (SEQ 3) and optssEchimera (SEQ 4) proteins for use as a diagnostic antigen in a diagnostic test for the detection of antibodies against Zika virus E protein. The commercially available monoclonal anti-E protein antibody of Zika virus (Anti-E-protein DIII (LR) [ZV-67], Absolute Antibody) was used as a positive control. The test sample constitute the mouse serum obtained after vaccination of Balb/c mice with Zika virus natural VLPs, consisting of prM and E proteins. The serum obtained after vaccination of Balb/c mice with PBS mixed with adjuvant was a negative control. The M-96 plate was coated overnight with an antigen - proteins precipitated from culture medium according to the method described in Example 1a. The protein concentration was 10 µg/ml per well (in 100 µl). Then, the blocking solution (3% bovine serum albumin in PBS buffer) was added (200 µl). After washing with PBS buffer with 0.05% Tween 20, 1/1000 dilution of serum or antibody was added in a volume of 100 µl and incubated 2 h. The plate was washed, and anti-mouse horseradish peroxidase (HRP)-conjugated secondary antibodies were added for 1 h. Antigen-antibody complexes were detected using tetramethylbenzidine substrate (TMB) (Life Technologies Inc USA). The reaction was then stopped by adding 0.5M sulfuric acid and read by reading the absorbance at 450 nm in a plate reader.

A similar absorbance value for the positive as well as for the tested samples was obtained. The value for tested sample was 3 times higher than the value obtained for the negative control, which confirm the usefulness of the recombinant optssE (SEQ 2), ssEchimera (SEQ 3) and optssEchimera (SEQ 4) proteins as antigens in the diagnostics (Fig. 5).

The tests initially showed the similar properties of other recombinant proteins based on SEQ 1 and VLPs based on SEQ 5-10 sequences as diagnostic antigens due to the presence of Zika virus prM protein and Zika virus cytoplasmic domain of E protein.

Sequence list - presented separately according to the standard.
SEQ. 1 - length 184 
Met Val Gly Val Ala Leu Ala Leu Ala Leu Ala Thr Thr Ala Met Ala
1               5                   10                  15
Ala Glu Val Thr Arg Arg Gly Ser Ala Tyr Tyr Met Tyr Leu Asp Arg
            20                  25                  30
Asn Asp Ala Gly Glu Ala Ile Ser Phe Pro Thr Thr Leu Gly Met Asn
        35                  40                  45
Lys Cys Tyr Ile Gln Ile Met Asp Leu Gly His Met Cys Asp Ala Thr
    50                  55                  60
Met Ser Tyr Glu Cys Pro Met Leu Asp Glu Gly Val Glu Pro Asp Asp
65                  70                  75                  80
Val Asp Cys Trp Cys Asn Thr Thr Ser Thr Trp Val Val Tyr Gly Thr
                85                  90                  95
Cys His His Lys Lys Gly Glu Ala Arg Arg Ser Arg Arg Ala Val Thr
            100                 105                 110
Leu Pro Ser His Ser Thr Arg Lys Leu Gln Thr Arg Ser Gln Thr Trp
        115                 120                 125
Leu Glu Ser Arg Glu Tyr Thr Lys His Leu Ile Arg Val Glu Asn Trp
    130                 135                 140
Ile Phe Arg Asn Pro Gly Phe Ala Leu Ala Ala Ala Ala Ile Ala Trp
145                 150                 155                 160
Leu Leu Gly Ser Ser Thr Ser Gln Lys Val Ile Tyr Leu Val Met Ile
                165                 170                 175
Leu Leu Ile Ala Pro Ala Tyr Ser
            180
SEQ. 2 - length 522 
Met Val Gly Val Ala Leu Ala Leu Ala Leu Ala Thr Thr Ala Met Ala
1               5                   10                  15
Ile Arg Cys Ile Gly Val Ser Asn Arg Asp Phe Val Glu Gly Met Ser
            20                  25                  30
Gly Gly Thr Trp Val Asp Val Val Leu Glu His Gly Gly Cys Val Thr
        35                  40                  45
Val Met Ala Gln Asp Lys Pro Thr Val Asp Ile Glu Leu Val Thr Thr
    50                  55                  60
Thr Val Ser Asn Met Ala Glu Val Arg Ser Tyr Cys Tyr Glu Ala Ser
65                  70                  75                  80
Ile Ser Asp Met Ala Ser Asp Ser Arg Cys Pro Thr Gln Gly Glu Ala
                85                  90                  95
Tyr Leu Asp Lys Gln Ser Asp Thr Gln Tyr Val Cys Lys Arg Thr Leu
            100                 105                 110
Val Asp Arg Gly Trp Gly Asn Gly Cys Gly Leu Phe Gly Lys Gly Ser
        115                 120                 125
Leu Val Thr Cys Ala Lys Phe Ala Cys Ser Lys Lys Met Thr Gly Lys
 
130                 135                 140
Ser Ile Gln Pro Glu Asn Leu Glu Tyr Arg Ile Met Leu Ser Val His
145                 150                 155                 160
Gly Ser Gln His Ser Gly Met Ile Val Asn Asp Thr Gly His Glu Thr
                165                 170                 175
Asp Glu Asn Arg Ala Lys Val Glu Ile Thr Pro Asn Ser Pro Arg Ala
            180                 185                 190
Glu Ala Thr Leu Gly Gly Phe Gly Ser Leu Gly Leu Asp Cys Glu Pro
        195                 200                 205
Arg Thr Gly Leu Asp Phe Ser Asp Leu Tyr Tyr Leu Thr Met Asn Asn
    210                 215                 220
Lys His Trp Leu Val His Lys Glu Trp Phe His Asp Ile Pro Leu Pro
225                 230                 235                 240
Trp His Ala Gly Ala Asp Thr Gly Thr Pro His Trp Asn Asn Lys Glu
                245                 250                 255
Ala Leu Val Glu Phe Lys Asp Ala His Ala Lys Arg Gln Thr Val Val
            260                 265                 270
Val Leu Gly Ser Gln Glu Gly Ala Val His Thr Ala Leu Ala Gly Ala
        275                 280                 285
Leu Glu Ala Glu Met Asp Gly Ala Lys Gly Arg Leu Ser Ser Gly His
    290                 295                 300
Leu Lys Cys Arg Leu Lys Met Asp Lys Leu Arg Leu Lys Gly Val Ser
305                 310                 315                 320
Tyr Ser Leu Cys Thr Ala Ala Phe Thr Phe Thr Lys Ile Pro Ala Glu
                325                 330                 335
Thr Leu His Gly Thr Val Thr Val Glu Val Gln Tyr Ala Gly Thr Asp
            340                 345                 350
Gly Pro Cys Lys Val Pro Ala Gln Met Ala Val Asp Met Gln Thr Leu
        355                 360                 365
Thr Pro Val Gly Arg Leu Ile Thr Ala Asn Pro Val Ile Thr Glu Ser
    370                 375                 380
Thr Glu Asn Ser Lys Met Met Leu Glu Leu Asp Pro Pro Phe Gly Asp
385                 390                 395                 400
Ser Tyr Ile Val Ile Gly Val Gly Glu Lys Lys Ile Thr His His Trp
                405                 410                 415
His Arg Ser Gly Ser Thr Ile Gly Lys Ala Phe Glu Ala Thr Val Arg
            420                 425                 430
Gly Ala Lys Arg Met Ala Val Leu Gly Asp Thr Ala Trp Asp Phe Gly
        435                 440                 445
Ser Val Gly Gly Ala Leu Asn Ser Leu Gly Lys Gly Ile His Gln Ile
    450                 455                 460
Phe Gly Ala Ala Phe Lys Ser Leu Phe Gly Gly Met Ser Trp Phe Ser
465                 470                 475                 480
Gln Ile Leu Ile Gly Thr Leu Leu Met Trp Leu Gly Leu Asn Thr Lys
                485                 490                 495
Asn Gly Ser Ile Ser Leu Met Cys Leu Ala Leu Gly Gly Val Leu Ile
            500                 505                 510
Phe Leu Ser Thr Ala Val Ser Ala Asp Val
        515                 520
SEQ. 3 - length 522 
Ser Gln Lys Val Ile Tyr Leu Val Met Ile Leu Leu Ile Ala Pro Ala
1               5                   10                  15
Tyr Ser Ile Arg Cys Ile Gly Val Ser Asn Arg Asp Phe Val Glu Gly
            20                  25                  30
Met Ser Gly Gly Thr Trp Val Asp Val Val Leu Glu His Gly Gly Cys
        35                  40                  45
Val Thr Val Met Ala Gln Asp Lys Pro Thr Val Asp Ile Glu Leu Val
    50                  55                  60
Thr Thr Thr Val Ser Asn Met Ala Glu Val Arg Ser Tyr Cys Tyr Glu
65                  70                  75                  80
Ala Ser Ile Ser Asp Met Ala Ser Asp Ser Arg Cys Pro Thr Gln Gly
                85                  90                  95
Glu Ala Tyr Leu Asp Lys Gln Ser Asp Thr Gln Tyr Val Cys Lys Arg
            100                 105                 110
Thr Leu Val Asp Arg Gly Trp Gly Asn Gly Cys Gly Leu Phe Gly Lys
        115                 120                 125
Gly Ser Leu Val Thr Cys Ala Lys Phe Ala Cys Ser Lys Lys Met Thr
    130                 135                 140
Gly Lys Ser Ile Gln Pro Glu Asn Leu Glu Tyr Arg Ile Met Leu Ser
145                 150                 155                 160
Val His Gly Ser Gln His Ser Gly Met Ile Val Asn Asp Thr Gly His
                165                 170                 175
Glu Thr Asp Glu Asn Arg Ala Lys Val Glu Ile Thr Pro Asn Ser Pro
            180                 185                 190
Arg Ala Glu Ala Thr Leu Gly Gly Phe Gly Ser Leu Gly Leu Asp Cys
        195                 200                 205
Glu Pro Arg Thr Gly Leu Asp Phe Ser Asp Leu Tyr Tyr Leu Thr Met
    210                 215                 220
Asn Asn Lys His Trp Leu Val His Lys Glu Trp Phe His Asp Ile Pro
225                 230                 235                 240
Leu Pro Trp His Ala Gly Ala Asp Thr Gly Thr Pro His Trp Asn Asn
                245                 250                 255
Lys Glu Ala Leu Val Glu Phe Lys Asp Ala His Ala Lys Arg Gln Thr
            260                 265                 270
Val Val Val Leu Gly Ser Gln Glu Gly Ala Val His Thr Ala Leu Ala
        275                 280                 285
Gly Ala Leu Glu Ala Glu Met Asp Gly Ala Lys Gly Arg Leu Ser Ser
    290                 295                 300
Gly His Leu Lys Cys Arg Leu Lys Met Asp Lys Leu Arg Leu Lys Gly
305                 310                 315                 320
Val Ser Tyr Ser Leu Cys Thr Ala Ala Phe Thr Phe Thr Lys Ile Pro
                325                 330                 335
Ala Glu Thr Leu His Gly Thr Val Thr Val Glu Val Gln Tyr Ala Gly
            340                 345                 350
Thr Asp Gly Pro Cys Lys Val Pro Ala Gln Met Ala Val Asp Met Gln
        355                 360                 365
Thr Leu Thr Pro Val Gly Arg Leu Ile Thr Ala Asn Pro Val Ile Thr
    370                 375                 380
Glu Ser Thr Glu Asn Ser Lys Met Met Leu Glu Leu Asp Pro Pro Phe
385                 390                 395                 400
Gly Asp Ser Tyr Ile Val Ile Gly Val Gly Glu Lys Lys Ile Thr His
                405                 410                 415
 
His Trp His Arg Ser Gly Ser Thr Ile Gly Arg Val Phe Gln Lys Thr
            420                 425                 430
Lys Lys Gly Ile Glu Arg Leu Thr Val Ile Gly Glu His Ala Trp Asp
        435                 440                 445
Phe Gly Ser Ala Gly Gly Phe Leu Ser Ser Ile Gly Lys Ala Val His
    450                 455                 460
Thr Val Leu Gly Gly Ala Phe Asn Ser Ile Phe Gly Gly Val Gly Phe
465                 470                 475                 480
Leu Pro Lys Leu Leu Leu Gly Val Ala Leu Ala Trp Leu Gly Leu Asn
                485                 490                 495
Met Arg Asn Pro Thr Met Ser Met Ser Phe Leu Leu Ala Gly Gly Leu
            500                 505                 510
Val Leu Ala Met Thr Leu Gly Val Gly Ala
        515                 520
SEQ. 4 - length 520 
Met Val Gly Val Ala Leu Ala Leu Ala Leu Ala Thr Thr Ala Met Ala
1               5                   10                  15
Ile Arg Cys Ile Gly Val Ser Asn Arg Asp Phe Val Glu Gly Met Ser
            20                  25                  30
Gly Gly Thr Trp Val Asp Val Val Leu Glu His Gly Gly Cys Val Thr
        35                  40                  45
Val Met Ala Gln Asp Lys Pro Thr Val Asp Ile Glu Leu Val Thr Thr
    50                  55                  60
Thr Val Ser Asn Met Ala Glu Val Arg Ser Tyr Cys Tyr Glu Ala Ser
65                  70                  75                  80
Ile Ser Asp Met Ala Ser Asp Ser Arg Cys Pro Thr Gln Gly Glu Ala
                85                  90                  95
Tyr Leu Asp Lys Gln Ser Asp Thr Gln Tyr Val Cys Lys Arg Thr Leu
            100                 105                 110
Val Asp Arg Gly Trp Gly Asn Gly Cys Gly Leu Phe Gly Lys Gly Ser
        115                 120                 125
Leu Val Thr Cys Ala Lys Phe Ala Cys Ser Lys Lys Met Thr Gly Lys
    130                 135                 140
Ser Ile Gln Pro Glu Asn Leu Glu Tyr Arg Ile Met Leu Ser Val His
145                 150                 155                 160
Gly Ser Gln His Ser Gly Met Ile Val Asn Asp Thr Gly His Glu Thr
                165                 170                 175
Asp Glu Asn Arg Ala Lys Val Glu Ile Thr Pro Asn Ser Pro Arg Ala
            180                 185                 190
Glu Ala Thr Leu Gly Gly Phe Gly Ser Leu Gly Leu Asp Cys Glu Pro
        195                 200                 205
Arg Thr Gly Leu Asp Phe Ser Asp Leu Tyr Tyr Leu Thr Met Asn Asn
    210                 215                 220
Lys His Trp Leu Val His Lys Glu Trp Phe His Asp Ile Pro Leu Pro
225                 230                 235                 240
Trp His Ala Gly Ala Asp Thr Gly Thr Pro His Trp Asn Asn Lys Glu
                245                 250                 255
Ala Leu Val Glu Phe Lys Asp Ala His Ala Lys Arg Gln Thr Val Val
            260                 265                 270
Val Leu Gly Ser Gln Glu Gly Ala Val His Thr Ala Leu Ala Gly Ala
        275                 280                 285
 
Leu Glu Ala Glu Met Asp Gly Ala Lys Gly Arg Leu Ser Ser Gly His
    290                 295                 300
Leu Lys Cys Arg Leu Lys Met Asp Lys Leu Arg Leu Lys Gly Val Ser
305                 310                 315                 320
Tyr Ser Leu Cys Thr Ala Ala Phe Thr Phe Thr Lys Ile Pro Ala Glu
                325                 330                 335
Thr Leu His Gly Thr Val Thr Val Glu Val Gln Tyr Ala Gly Thr Asp
            340                 345                 350
Gly Pro Cys Lys Val Pro Ala Gln Met Ala Val Asp Met Gln Thr Leu
        355                 360                 365
Thr Pro Val Gly Arg Leu Ile Thr Ala Asn Pro Val Ile Thr Glu Ser
    370                 375                 380
Thr Glu Asn Ser Lys Met Met Leu Glu Leu Asp Pro Pro Phe Gly Asp
385                 390                 395                 400
Ser Tyr Ile Val Ile Gly Val Gly Glu Lys Lys Ile Thr His His Trp
                405                 410                 415
His Arg Ser Gly Ser Thr Ile Gly Arg Val Phe Gln Lys Thr Lys Lys
            420                 425                 430
Gly Ile Glu Arg Leu Thr Val Ile Gly Glu His Ala Trp Asp Phe Gly
        435                 440                 445
Ser Ala Gly Gly Phe Leu Ser Ser Ile Gly Lys Ala Val His Thr Val
    450                 455                 460
Leu Gly Gly Ala Phe Asn Ser Ile Phe Gly Gly Val Gly Phe Leu Pro
465                 470                 475                 480
Lys Leu Leu Leu Gly Val Ala Leu Ala Trp Leu Gly Leu Asn Met Arg
                485                 490                 495
Asn Pro Thr Met Ser Met Ser Phe Leu Leu Ala Gly Gly Leu Val Leu
            500                 505                 510
Ala Met Thr Leu Gly Val Gly Ala
        515                 520
SEQ. 5 - length 690 
Met Val Gly Val Ala Leu Ala Leu Ala Leu Ala Thr Thr Ala Met Ala
1               5                   10                  15
Ala Glu Val Thr Arg Arg Gly Ser Ala Tyr Tyr Met Tyr Leu Asp Arg
            20                  25                  30
Asn Asp Ala Gly Glu Ala Ile Ser Phe Pro Thr Thr Leu Gly Met Asn
        35                  40                  45
Lys Cys Tyr Ile Gln Ile Met Asp Leu Gly His Met Cys Asp Ala Thr
    50                  55                  60
Met Ser Tyr Glu Cys Pro Met Leu Asp Glu Gly Val Glu Pro Asp Asp
65                  70                  75                  80
Val Asp Cys Trp Cys Asn Thr Thr Ser Thr Trp Val Val Tyr Gly Thr
                85                  90                  95
Cys His His Lys Lys Gly Glu Ala Arg Arg Ser Arg Arg Ala Val Thr
            100                 105                 110
Leu Pro Ser His Ser Thr Arg Lys Leu Gln Thr Arg Ser Gln Thr Trp
        115                 120                 125
Leu Glu Ser Arg Glu Tyr Thr Lys His Leu Ile Arg Val Glu Asn Trp
    130                 135                 140
 
Ile Phe Arg Asn Pro Gly Phe Ala Leu Ala Ala Ala Ile Ala Trp
145                 150                 155                 160
Leu Leu Gly Ser Ser Thr Ser Gln Lys Val Ile Tyr Leu Val Met Ile
                165                 170                 175
Leu Leu Ile Ala Pro Ala Tyr Ser Ile Arg Cys Ile Gly Val Ser Asn
            180                 185                 190
Arg Asp Phe Val Glu Gly Met Ser Gly Gly Thr Trp Val Asp Val Val
        195                 200                 205
Leu Glu His Gly Gly Cys Val Thr Val Met Ala Gln Asp Lys Pro Thr
    210                 215                 220
Val Asp Ile Glu Leu Val Thr Thr Thr Val Ser Asn Met Ala Glu Val
225                 230                 235                 240
Arg Ser Tyr Cys Tyr Glu Ala Ser Ile Ser Asp Met Ala Ser Asp Ser
                245                 250                 255
Arg Cys Pro Thr Gln Gly Glu Ala Tyr Leu Asp Lys Gln Ser Asp Thr
            260                 265                 270
Gln Tyr Val Cys Lys Arg Thr Leu Val Asp Arg Gly Trp Gly Asn Gly
        275                 280                 285
Cys Gly Leu Phe Gly Lys Gly Ser Leu Val Thr Cys Ala Lys Phe Ala
    290                 295                 300
Cys Ser Lys Lys Met Thr Gly Lys Ser Ile Gln Pro Glu Asn Leu Glu
305                 310                 315                 320
Tyr Arg Ile Met Leu Ser Val His Gly Ser Gln His Ser Gly Met Ile
                325                 330                 335
Val Asn Asp Thr Gly His Glu Thr Asp Glu Asn Arg Ala Lys Val Glu
            340                 345                 350
Ile Thr Pro Asn Ser Pro Arg Ala Glu Ala Thr Leu Gly Gly Phe Gly
        355                 360                 365
Ser Leu Gly Leu Asp Cys Glu Pro Arg Thr Gly Leu Asp Phe Ser Asp
    370                 375                 380
Leu Tyr Tyr Leu Thr Met Asn Asn Lys His Trp Leu Val His Lys Glu
385                 390                 395                 400
Trp Phe His Asp Ile Pro Leu Pro Trp His Ala Gly Ala Asp Thr Gly
                405                 410                 415
Thr Pro His Trp Asn Asn Lys Glu Ala Leu Val Glu Phe Lys Asp Ala
            420                 425                 430
His Ala Lys Arg Gln Thr Val Val Val Leu Gly Ser Gln Glu Gly Ala
        435                 440                 445
Val His Thr Ala Leu Ala Gly Ala Leu Glu Ala Glu Met Asp Gly Ala
    450                 455                 460
Lys Gly Arg Leu Ser Ser Gly His Leu Lys Cys Arg Leu Lys Met Asp
465                 470                 475                 480
Lys Leu Arg Leu Lys Gly Val Ser Tyr Ser Leu Cys Thr Ala Ala Phe
                485                 490                 495
Thr Phe Thr Lys Ile Pro Ala Glu Thr Leu His Gly Thr Val Thr Val
            500                 505                 510
Glu Val Gln Tyr Ala Gly Thr Asp Gly Pro Cys Lys Val Pro Ala Gln
        515                 520                 525
Met Ala Val Asp Met Gln Thr Leu Thr Pro Val Gly Arg Leu Ile Thr
    530                 535                 540
Ala Asn Pro Val Ile Thr Glu Ser Thr Glu Asn Ser Lys Met Met Leu
545                 550                 555                 560
Glu Leu Asp Pro Pro Phe Gly Asp Ser Tyr Ile Val Ile Gly Val Gly
                565                 570                 575
 
Glu Lys Lys Ile Thr His His Trp His Arg Ser Gly Ser Thr Ile Gly
            580                 585                 590
Lys Ala Phe Glu Ala Thr Val Arg Gly Ala Lys Arg Met Ala Val Leu
        595                 600                 605
Gly Asp Thr Ala Trp Asp Phe Gly Ser Val Gly Gly Ala Leu Asn Ser
    610                 615                 620
Leu Gly Lys Gly Ile His Gln Ile Phe Gly Ala Ala Phe Lys Ser Leu
625                 630                 635                 640
Phe Gly Gly Met Ser Trp Phe Ser Gln Ile Leu Ile Gly Thr Leu Leu
                645                 650                 655
Met Trp Leu Gly Leu Asn Thr Lys Asn Gly Ser Ile Ser Leu Met Cys
            660                 665                 670
Leu Ala Leu Gly Gly Val Leu Ile Phe Leu Ser Thr Ala Val Ser Ala
        675                 680                 685
Asp Val
    690
SEQ. 6 - length 688 
Met Val Gly Val Ala Leu Ala Leu Ala Leu Ala Thr Thr Ala Met Ala
1               5                   10                  15
Ala Glu Val Thr Arg Arg Gly Ser Ala Tyr Tyr Met Tyr Leu Asp Arg
            20                  25                  30
Asn Asp Ala Gly Glu Ala Ile Ser Phe Pro Thr Thr Leu Gly Met Asn
        35                  40                  45
Lys Cys Tyr Ile Gln Ile Met Asp Leu Gly His Met Cys Asp Ala Thr
    50                  55                  60
Met Ser Tyr Glu Cys Pro Met Leu Asp Glu Gly Val Glu Pro Asp Asp
65                  70                  75                  80
Val Asp Cys Trp Cys Asn Thr Thr Ser Thr Trp Val Val Tyr Gly Thr
                85                  90                  95
Cys His His Lys Lys Gly Glu Ala Arg Arg Ser Arg Arg Ala Val Thr
            100                 105                 110
Leu Pro Ser His Ser Thr Arg Lys Leu Gln Thr Arg Ser Gln Thr Trp
        115                 120                 125
Leu Glu Ser Arg Glu Tyr Thr Lys His Leu Ile Arg Val Glu Asn Trp
    130                 135                 140
Ile Phe Arg Asn Pro Gly Phe Ala Leu Ala Ala Ala Ala Ile Ala Trp
145                 150                 155                 160
Leu Leu Gly Ser Ser Thr Ser Gln Lys Val Ile Tyr Leu Val Met Ile
                165                 170                 175
Leu Leu Ile Ala Pro Ala Tyr Ser Ile Arg Cys Ile Gly Val Ser Asn
            180                 185                 190
Arg Asp Phe Val Glu Gly Met Ser Gly Gly Thr Trp Val Asp Val Val
        195                 200                 205
Leu Glu His Gly Gly Cys Val Thr Val Met Ala Gln Asp Lys Pro Thr
    210                 215                 220
Val Asp Ile Glu Leu Val Thr Thr Thr Val Ser Asn Met Ala Glu Val
225                 230                 235                 240
Arg Ser Tyr Cys Tyr Glu Ala Ser Ile Ser Asp Met Ala Ser Asp Ser
                245                 250                 255
 
Arg Cys Pro Thr Gln Gly Glu Ala Tyr Leu Asp Lys Gln Ser Asp Thr
            260                 265                 270
Gln Tyr Val Cys Lys Arg Thr Leu Val Asp Arg Gly Trp Gly Asn Gly
        275                 280                 285
Cys Gly Leu Phe Gly Lys Gly Ser Leu Val Thr Cys Ala Lys Phe Ala
    290                 295                 300
Cys Ser Lys Lys Met Thr Gly Lys Ser Ile Gln Pro Glu Asn Leu Glu
305                 310                 315                 320
Tyr Arg Ile Met Leu Ser Val His Gly Ser Gln His Ser Gly Met Ile
                325                 330                 335
Val Asn Asp Thr Gly His Glu Thr Asp Glu Asn Arg Ala Lys Val Glu
            340                 345                 350
Ile Thr Pro Asn Ser Pro Arg Ala Glu Ala Thr Leu Gly Gly Phe Gly
        355                 360                 365
Ser Leu Gly Leu Asp Cys Glu Pro Arg Thr Gly Leu Asp Phe Ser Asp
    370                 375                 380
Leu Tyr Tyr Leu Thr Met Asn Asn Lys His Trp Leu Val His Lys Glu
385                 390                 395                 400
Trp Phe His Asp Ile Pro Leu Pro Trp His Ala Gly Ala Asp Thr Gly
                405                 410                 415
Thr Pro His Trp Asn Asn Lys Glu Ala Leu Val Glu Phe Lys Asp Ala
            420                 425                 430
His Ala Lys Arg Gln Thr Val Val Val Leu Gly Ser Gln Glu Gly Ala
        435                 440                 445
Val His Thr Ala Leu Ala Gly Ala Leu Glu Ala Glu Met Asp Gly Ala
    450                 455                 460
Lys Gly Arg Leu Ser Ser Gly His Leu Lys Cys Arg Leu Lys Met Asp
465                 470                 475                 480
Lys Leu Arg Leu Lys Gly Val Ser Tyr Ser Leu Cys Thr Ala Ala Phe
                485                 490                 495
Thr Phe Thr Lys Ile Pro Ala Glu Thr Leu His Gly Thr Val Thr Val
            500                 505                 510
Glu Val Gln Tyr Ala Gly Thr Asp Gly Pro Cys Lys Val Pro Ala Gln
        515                 520                 525
Met Ala Val Asp Met Gln Thr Leu Thr Pro Val Gly Arg Leu Ile Thr
    530                 535                 540
Ala Asn Pro Val Ile Thr Glu Ser Thr Glu Asn Ser Lys Met Met Leu
545                 550                 555                 560
Glu Leu Asp Pro Pro Phe Gly Asp Ser Tyr Ile Val Ile Gly Val Gly
                565                 570                 575
Glu Lys Lys Ile Thr His His Trp His Arg Ser Gly Ser Thr Ile Gly
            580                 585                 590
Arg Val Phe Gln Lys Thr Lys Lys Gly Ile Glu Arg Leu Thr Val Ile
        595                 600                 605
Gly Glu His Ala Trp Asp Phe Gly Ser Ala Gly Gly Phe Leu Ser Ser
    610                 615                 620
Ile Gly Lys Ala Val His Thr Val Leu Gly Gly Ala Phe Asn Ser Ile
625                 630                 635                 640
Phe Gly Gly Val Gly Phe Leu Pro Lys Leu Leu Leu Gly Val Ala Leu
                645                 650                 655
Ala Trp Leu Gly Leu Asn Met Arg Asn Pro Thr Met Ser Met Ser Phe
            660                 665                 670
Leu Leu Ala Gly Gly Leu Val Leu Ala Met Thr Leu Gly Val Gly Ala
        675                 680                 685
SEQ. 7 - length 732 
Gly Ala Asp Thr Ser Val Gly Ile Val Gly Leu Leu Leu Thr Thr Ala
1               5                   10                  15
Met Ala Ala Glu Val Thr Arg Arg Gly Ser Ala Tyr Tyr Met Tyr Leu
            20                  25                  30
Asp Arg Asn Asp Ala Gly Glu Ala Ile Ser Phe Pro Thr Thr Leu Gly
        35                  40                  45
Met Asn Lys Cys Tyr Ile Gln Ile Met Asp Leu Gly His Met Cys Asp
    50                  55                  60
Ala Thr Met Ser Tyr Glu Cys Pro Met Leu Asp Glu Gly Val Glu Pro
65                  70                  75                  80
Asp Asp Val Asp Cys Trp Cys Asn Thr Thr Ser Thr Trp Val Val Tyr
                85                  90                  95
Gly Thr Cys His His Lys Lys Gly Glu Ala Arg Arg Ser Arg Arg Ala
            100                 105                 110
Val Thr Leu Pro Ser His Ser Thr Arg Lys Leu Gln Thr Arg Ser Gln
        115                 120                 125
Thr Trp Leu Glu Ser Arg Glu Tyr Thr Lys His Leu Ile Arg Val Glu
    130                 135                 140
Asn Trp Ile Phe Arg Asn Pro Gly Phe Ala Leu Ala Ala Ala Ala Ile
145                 150                 155                 160
Ala Trp Leu Leu Gly Ser Ser Thr Ser Gln Lys Val Ile Tyr Leu Val
                165                 170                 175
Met Ile Leu Leu Ile Ala Pro Ala Tyr Ser Gly Ser Gly Ala Thr Asn
            180                 185                 190
Phe Ser Leu Leu Lys Gln Ala Gly Asp Val Glu Glu Asn Pro Gly Pro
        195                 200                 205
Ser Gln Lys Val Ile Tyr Leu Val Met Ile Leu Leu Ile Ala Pro Ala
    210                 215                 220
Tyr Ser Ile Arg Cys Ile Gly Val Ser Asn Arg Asp Phe Val Glu Gly
225                 230                 235                 240
Met Ser Gly Gly Thr Trp Val Asp Val Val Leu Glu His Gly Gly Cys
                245                 250                 255
Val Thr Val Met Ala Gln Asp Lys Pro Thr Val Asp Ile Glu Leu Val
            260                 265                 270
Thr Thr Thr Val Ser Asn Met Ala Glu Val Arg Ser Tyr Cys Tyr Glu
        275                 280                 285
Ala Ser Ile Ser Asp Met Ala Ser Asp Ser Arg Cys Pro Thr Gln Gly
    290                 295                 300
Glu Ala Tyr Leu Asp Lys Gln Ser Asp Thr Gln Tyr Val Cys Lys Arg
305                 310                 315                 320
Thr Leu Val Asp Arg Gly Trp Gly Asn Gly Cys Gly Leu Phe Gly Lys
                325                 330                 335
Gly Ser Leu Val Thr Cys Ala Lys Phe Ala Cys Ser Lys Lys Met Thr
            340                 345                 350
Gly Lys Ser Ile Gln Pro Glu Asn Leu Glu Tyr Arg Ile Met Leu Ser
        355                 360                 365
Val His Gly Ser Gln His Ser Gly Met Ile Val Asn Asp Thr Gly His
    370                 375                 380
Glu Thr Asp Glu Asn Arg Ala Lys Val Glu Ile Thr Pro Asn Ser Pro
385                 390                 395                 400
 
Arg Ala Glu Ala Thr Leu Gly Gly Phe Gly Ser Leu Gly Leu Asp Cys
                405                 410                 415
Glu Pro Arg Thr Gly Leu Asp Phe Ser Asp Leu Tyr Tyr Leu Thr Met
            420                 425                 430
Asn Asn Lys His Trp Leu Val His Lys Glu Trp Phe His Asp Ile Pro
        435                 440                 445
Leu Pro Trp His Ala Gly Ala Asp Thr Gly Thr Pro His Trp Asn Asn
    450                 455                 460
Lys Glu Ala Leu Val Glu Phe Lys Asp Ala His Ala Lys Arg Gln Thr
465                 470                 475                 480
Val Val Val Leu Gly Ser Gln Glu Gly Ala Val His Thr Ala Leu Ala
                485                 490                 495
Gly Ala Leu Glu Ala Glu Met Asp Gly Ala Lys Gly Arg Leu Ser Ser
            500                 505                 510
Gly His Leu Lys Cys Arg Leu Lys Met Asp Lys Leu Arg Leu Lys Gly
        515                 520                 525
Val Ser Tyr Ser Leu Cys Thr Ala Ala Phe Thr Phe Thr Lys Ile Pro
    530                 535                 540
Ala Glu Thr Leu His Gly Thr Val Thr Val Glu Val Gln Tyr Ala Gly
545                 550                 555                 560
Thr Asp Gly Pro Cys Lys Val Pro Ala Gln Met Ala Val Asp Met Gln
                565                 570                 575
Thr Leu Thr Pro Val Gly Arg Leu Ile Thr Ala Asn Pro Val Ile Thr
            580                 585                 590
Glu Ser Thr Glu Asn Ser Lys Met Met Leu Glu Leu Asp Pro Pro Phe
        595                 600                 605
Gly Asp Ser Tyr Ile Val Ile Gly Val Gly Glu Lys Lys Ile Thr His
    610                 615                 620
His Trp His Arg Ser Gly Ser Thr Ile Gly Lys Ala Phe Glu Ala Thr
625                 630                 635                 640
Val Arg Gly Ala Lys Arg Met Ala Val Leu Gly Asp Thr Ala Trp Asp
                645                 650                 655
Phe Gly Ser Val Gly Gly Ala Leu Asn Ser Leu Gly Lys Gly Ile His
            660                 665                 670
Gln Ile Phe Gly Ala Ala Phe Lys Ser Leu Phe Gly Gly Met Ser Trp
        675                 680                 685
Phe Ser Gln Ile Leu Ile Gly Thr Leu Leu Met Trp Leu Gly Leu Asn
    690                 695                 700
Thr Lys Asn Gly Ser Ile Ser Leu Met Cys Leu Ala Leu Gly Gly Val
705                 710                 715                 720
Leu Ile Phe Leu Ser Thr Ala Val Ser Ala Asp Val
                725                 730
SEQ. 8 - length 730 
Gly Ala Asp Thr Ser Val Gly Ile Val Gly Leu Leu Leu Thr Thr Ala
1               5                   10                  15
Met Ala Ala Glu Val Thr Arg Arg Gly Ser Ala Tyr Tyr Met Tyr Leu
            20                  25                  30
Asp Arg Asn Asp Ala Gly Glu Ala Ile Ser Phe Pro Thr Thr Leu Gly
        35                  40                  45
 
Met Asn Lys Cys Tyr Ile Gln Ile Met Asp Leu Gly His Met Cys Asp
    50                  55                  60
Ala Thr Met Ser Tyr Glu Cys Pro Met Leu Asp Glu Gly Val Glu Pro
65                  70                  75                  80
Asp Asp Val Asp Cys Trp Cys Asn Thr Thr Ser Thr Trp Val Val Tyr
                85                  90                  95
Gly Thr Cys His His Lys Lys Gly Glu Ala Arg Arg Ser Arg Arg Ala
            100                 105                 110
Val Thr Leu Pro Ser His Ser Thr Arg Lys Leu Gln Thr Arg Ser Gln
        115                 120                 125
Thr Trp Leu Glu Ser Arg Glu Tyr Thr Lys His Leu Ile Arg Val Glu
    130                 135                 140
Asn Trp Ile Phe Arg Asn Pro Gly Phe Ala Leu Ala Ala Ala Ala Ile
145                 150                 155                 160
Ala Trp Leu Leu Gly Ser Ser Thr Ser Gln Lys Val Ile Tyr Leu Val
                165                 170                 175
Met Ile Leu Leu Ile Ala Pro Ala Tyr Ser Gly Ser Gly Ala Thr Asn
            180                 185                 190
Phe Ser Leu Leu Lys Gln Ala Gly Asp Val Glu Glu Asn Pro Gly Pro
        195                 200                 205
Ser Gln Lys Val Ile Tyr Leu Val Met Ile Leu Leu Ile Ala Pro Ala
    210                 215                 220
Tyr Ser Ile Arg Cys Ile Gly Val Ser Asn Arg Asp Phe Val Glu Gly
225                 230                 235                 240
Met Ser Gly Gly Thr Trp Val Asp Val Val Leu Glu His Gly Gly Cys
                245                 250                 255
Val Thr Val Met Ala Gln Asp Lys Pro Thr Val Asp Ile Glu Leu Val
            260                 265                 270
Thr Thr Thr Val Ser Asn Met Ala Glu Val Arg Ser Tyr Cys Tyr Glu
        275                 280                 285
Ala Ser Ile Ser Asp Met Ala Ser Asp Ser Arg Cys Pro Thr Gln Gly
    290                 295                 300
Glu Ala Tyr Leu Asp Lys Gln Ser Asp Thr Gln Tyr Val Cys Lys Arg
305                 310                 315                 320
Thr Leu Val Asp Arg Gly Trp Gly Asn Gly Cys Gly Leu Phe Gly Lys
                325                 330                 335
Gly Ser Leu Val Thr Cys Ala Lys Phe Ala Cys Ser Lys Lys Met Thr
            340                 345                 350
Gly Lys Ser Ile Gln Pro Glu Asn Leu Glu Tyr Arg Ile Met Leu Ser
        355                 360                 365
Val His Gly Ser Gln His Ser Gly Met Ile Val Asn Asp Thr Gly His
    370                 375                 380
Glu Thr Asp Glu Asn Arg Ala Lys Val Glu Ile Thr Pro Asn Ser Pro
385                 390                 395                 400
Arg Ala Glu Ala Thr Leu Gly Gly Phe Gly Ser Leu Gly Leu Asp Cys
                405                 410                 415
Glu Pro Arg Thr Gly Leu Asp Phe Ser Asp Leu Tyr Tyr Leu Thr Met
            420                 425                 430
Asn Asn Lys His Trp Leu Val His Lys Glu Trp Phe His Asp Ile Pro
        435                 440                 445
Leu Pro Trp His Ala Gly Ala Asp Thr Gly Thr Pro His Trp Asn Asn
    450                 455                 460
Lys Glu Ala Leu Val Glu Phe Lys Asp Ala His Ala Lys Arg Gln Thr
465                 470                 475                 480
 
Val Val Val Leu Gly Ser Gln Glu Gly Ala Val His Thr Ala Leu Ala
                485                 490                 495
Gly Ala Leu Glu Ala Glu Met Asp Gly Ala Lys Gly Arg Leu Ser Ser
            500                 505                 510
Gly His Leu Lys Cys Arg Leu Lys Met Asp Lys Leu Arg Leu Lys Gly
        515                 520                 525
Val Ser Tyr Ser Leu Cys Thr Ala Ala Phe Thr Phe Thr Lys Ile Pro
    530                 535                 540
Ala Glu Thr Leu His Gly Thr Val Thr Val Glu Val Gln Tyr Ala Gly
545                 550                 555                 560
Thr Asp Gly Pro Cys Lys Val Pro Ala Gln Met Ala Val Asp Met Gln
                565                 570                 575
Thr Leu Thr Pro Val Gly Arg Leu Ile Thr Ala Asn Pro Val Ile Thr
            580                 585                 590
Glu Ser Thr Glu Asn Ser Lys Met Met Leu Glu Leu Asp Pro Pro Phe
        595                 600                 605
Gly Asp Ser Tyr Ile Val Ile Gly Val Gly Glu Lys Lys Ile Thr His
    610                 615                 620
His Trp His Arg Ser Gly Ser Thr Ile Gly Arg Val Phe Gln Lys Thr
625                 630                 635                 640
Lys Lys Gly Ile Glu Arg Leu Thr Val Ile Gly Glu His Ala Trp Asp
                645                 650                 655
Phe Gly Ser Ala Gly Gly Phe Leu Ser Ser Ile Gly Lys Ala Val His
            660                 665                 670
Thr Val Leu Gly Gly Ala Phe Asn Ser Ile Phe Gly Gly Val Gly Phe
        675                 680                 685
Leu Pro Lys Leu Leu Leu Gly Val Ala Leu Ala Trp Leu Gly Leu Asn
    690                 695                 700
Met Arg Asn Pro Thr Met Ser Met Ser Phe Leu Leu Ala Gly Gly Leu
705                 710                 715                 720
Val Leu Ala Met Thr Leu Gly Val Gly Ala
                725                 730
SEQ.9 - length 730 
Met Val Gly Val Ala Leu Ala Leu Ala Leu Ala Thr Thr Ala Met Ala
1               5                   10                  15
Ala Glu Val Thr Arg Arg Gly Ser Ala Tyr Tyr Met Tyr Leu Asp Arg
            20                  25                  30
Asn Asp Ala Gly Glu Ala Ile Ser Phe Pro Thr Thr Leu Gly Met Asn
        35                  40                  45
Lys Cys Tyr Ile Gln Ile Met Asp Leu Gly His Met Cys Asp Ala Thr
    50                  55                  60
Met Ser Tyr Glu Cys Pro Met Leu Asp Glu Gly Val Glu Pro Asp Asp
65                  70                  75                  80
Val Asp Cys Trp Cys Asn Thr Thr Ser Thr Trp Val Val Tyr Gly Thr
                85                  90                  95
Cys His His Lys Lys Gly Glu Ala Arg Arg Ser Arg Arg Ala Val Thr
            100                 105                 110
Leu Pro Ser His Ser Thr Arg Lys Leu Gln Thr Arg Ser Gln Thr Trp
        115                 120                 125
 
Leu Glu Ser Arg Glu Tyr Thr Lys His Leu Ile Arg Val Glu Asn Trp
        130                 135                 140
Ile Phe Arg Asn Pro Gly Phe Ala Leu Ala Ala Ala Ile Ala Trp
145                 150                 155                 160
Leu Leu Gly Ser Ser Thr Ser Gln Lys Val Ile Tyr Leu Val Met Ile
                165                 170                 175
Leu Leu Ile Ala Pro Ala Tyr Ser Gly Ser Gly Ala Thr Asn Phe Ser
            180                 185                 190
Leu Leu Lys Gln Ala Gly Asp Val Glu Glu Asn Pro Gly Pro Ser Gln
        195                 200                 205
Lys Val Ile Tyr Leu Val Met Ile Leu Leu Ile Ala Pro Ala Tyr Ser
    210                 215                 220
Ile Arg Cys Ile Gly Val Ser Asn Arg Asp Phe Val Glu Gly Met Ser
225                 230                 235                 240
Gly Gly Thr Trp Val Asp Val Val Leu Glu His Gly Gly Cys Val Thr
                245                 250                 255
Val Met Ala Gln Asp Lys Pro Thr Val Asp Ile Glu Leu Val Thr Thr
            260                 265                 270
Thr Val Ser Asn Met Ala Glu Val Arg Ser Tyr Cys Tyr Glu Ala Ser
        275                 280                 285
Ile Ser Asp Met Ala Ser Asp Ser Arg Cys Pro Thr Gln Gly Glu Ala
    290                 295                 300
Tyr Leu Asp Lys Gln Ser Asp Thr Gln Tyr Val Cys Lys Arg Thr Leu
305                 310                 315                 320
Val Asp Arg Gly Trp Gly Asn Gly Cys Gly Leu Phe Gly Lys Gly Ser
                325                 330                 335
Leu Val Thr Cys Ala Lys Phe Ala Cys Ser Lys Lys Met Thr Gly Lys
            340                 345                 350
Ser Ile Gln Pro Glu Asn Leu Glu Tyr Arg Ile Met Leu Ser Val His
        355                 360                 365
Gly Ser Gln His Ser Gly Met Ile Val Asn Asp Thr Gly His Glu Thr
    370                 375                 380
Asp Glu Asn Arg Ala Lys Val Glu Ile Thr Pro Asn Ser Pro Arg Ala
385                 390                 395                 400
Glu Ala Thr Leu Gly Gly Phe Gly Ser Leu Gly Leu Asp Cys Glu Pro
                405                 410                 415
Arg Thr Gly Leu Asp Phe Ser Asp Leu Tyr Tyr Leu Thr Met Asn Asn
            420                 425                 430
Lys His Trp Leu Val His Lys Glu Trp Phe His Asp Ile Pro Leu Pro
        435                 440                 445
Trp His Ala Gly Ala Asp Thr Gly Thr Pro His Trp Asn Asn Lys Glu
    450                 455                 460
Ala Leu Val Glu Phe Lys Asp Ala His Ala Lys Arg Gln Thr Val Val
465                 470                 475                 480
Val Leu Gly Ser Gln Glu Gly Ala Val His Thr Ala Leu Ala Gly Ala
                485                 490                 495
Leu Glu Ala Glu Met Asp Gly Ala Lys Gly Arg Leu Ser Ser Gly His
            500                 505                 510
Leu Lys Cys Arg Leu Lys Met Asp Lys Leu Arg Leu Lys Gly Val Ser
        515                 520                 525
Tyr Ser Leu Cys Thr Ala Ala Phe Thr Phe Thr Lys Ile Pro Ala Glu
    530                 535                 540
Thr Leu His Gly Thr Val Thr Val Glu Val Gln Tyr Ala Gly Thr Asp
545                 550                 555                 560
 
Gly Pro Cys Lys Val Pro Ala Gln Met Ala Val Asp Met Gln Thr Leu
                565                 570                 575
Thr Pro Val Gly Arg Leu Ile Thr Ala Asn Pro Val Ile Thr Glu Ser
            580                 585                 590
Thr Glu Asn Ser Lys Met Met Leu Glu Leu Asp Pro Pro Phe Gly Asp
        595                 600                 605
Ser Tyr Ile Val Ile Gly Val Gly Glu Lys Lys Ile Thr His His Trp
    610                 615                 620
His Arg Ser Gly Ser Thr Ile Gly Lys Ala Phe Glu Ala Thr Val Arg
625                 630                 635                 640
Gly Ala Lys Arg Met Ala Val Leu Gly Asp Thr Ala Trp Asp Phe Gly
                645                 650                 655
Ser Val Gly Gly Ala Leu Asn Ser Leu Gly Lys Gly Ile His Gln Ile
            660                 665                 670
Phe Gly Ala Ala Phe Lys Ser Leu Phe Gly Gly Met Ser Trp Phe Ser
        675                 680                 685
Gln Ile Leu Ile Gly Thr Leu Leu Met Trp Leu Gly Leu Asn Thr Lys
    690                 695                 700
Asn Gly Ser Ile Ser Leu Met Cys Leu Ala Leu Gly Gly Val Leu Ile
705                 710                 715                 720
Phe Leu Ser Thr Ala Val Ser Ala Asp Val
                725                 730
SEQ. 10 - length 728 
Met Val Gly Val Ala Leu Ala Leu Ala Leu Ala Thr Thr Ala Met Ala
1               5                   10                  15
Ala Glu Val Thr Arg Arg Gly Ser Ala Tyr Tyr Met Tyr Leu Asp Arg
            20                  25                  30
Asn Asp Ala Gly Glu Ala Ile Ser Phe Pro Thr Thr Leu Gly Met Asn
        35                  40                  45
Lys Cys Tyr Ile Gln Ile Met Asp Leu Gly His Met Cys Asp Ala Thr
    50                  55                  60
Met Ser Tyr Glu Cys Pro Met Leu Asp Glu Gly Val Glu Pro Asp Asp
65                  70                  75                  80
Val Asp Cys Trp Cys Asn Thr Thr Ser Thr Trp Val Val Tyr Gly Thr
                85                  90                  95
Cys His His Lys Lys Gly Glu Ala Arg Arg Ser Arg Arg Ala Val Thr
            100                 105                 110
Leu Pro Ser His Ser Thr Arg Lys Leu Gln Thr Arg Ser Gln Thr Trp
        115                 120                 125
Leu Glu Ser Arg Glu Tyr Thr Lys His Leu Ile Arg Val Glu Asn Trp
    130                 135                 140
Ile Phe Arg Asn Pro Gly Phe Ala Leu Ala Ala Ala Ala Ile Ala Trp
145                 150                 155                 160
Leu Leu Gly Ser Ser Thr Ser Gln Lys Val Ile Tyr Leu Val Met Ile
                165                 170                 175
Leu Leu Ile Ala Pro Ala Tyr Ser Gly Ser Gly Ala Thr Asn Phe Ser
            180                 185                 190
Leu Leu Lys Gln Ala Gly Asp Val Glu Glu Asn Pro Gly Pro Ser Gln
        195                 200                 205
 
Lys Val Ile Tyr Leu Val Met Ile Leu Leu Ile Ala Pro Ala Tyr Ser
    210                 215                 220
Ile Arg Cys Ile Gly Val Ser Asn Arg Asp Phe Val Glu Gly Met Ser
225                 230                 235                 240
Gly Gly Thr Trp Val Asp Val Val Leu Glu His Gly Gly Cys Val Thr
                245                 250                 255
Val Met Ala Gln Asp Lys Pro Thr Val Asp Ile Glu Leu Val Thr Thr
            260                 265                 270
Thr Val Ser Asn Met Ala Glu Val Arg Ser Tyr Cys Tyr Glu Ala Ser
        275                 280                 285
Ile Ser Asp Met Ala Ser Asp Ser Arg Cys Pro Thr Gln Gly Glu Ala
    290                 295                 300
Tyr Leu Asp Lys Gln Ser Asp Thr Gln Tyr Val Cys Lys Arg Thr Leu
305                 310                 315                 320
Val Asp Arg Gly Trp Gly Asn Gly Cys Gly Leu Phe Gly Lys Gly Ser
                325                 330                 335
Leu Val Thr Cys Ala Lys Phe Ala Cys Ser Lys Lys Met Thr Gly Lys
            340                 345                 350
Ser Ile Gln Pro Glu Asn Leu Glu Tyr Arg Ile Met Leu Ser Val His
        355                 360                 365
Gly Ser Gln His Ser Gly Met Ile Val Asn Asp Thr Gly His Glu Thr
    370                 375                 380
Asp Glu Asn Arg Ala Lys Val Glu Ile Thr Pro Asn Ser Pro Arg Ala
385                 390                 395                 400
Glu Ala Thr Leu Gly Gly Phe Gly Ser Leu Gly Leu Asp Cys Glu Pro
                405                 410                 415
Arg Thr Gly Leu Asp Phe Ser Asp Leu Tyr Tyr Leu Thr Met Asn Asn
            420                 425                 430
Lys His Trp Leu Val His Lys Glu Trp Phe His Asp Ile Pro Leu Pro
        435                 440                 445
Trp His Ala Gly Ala Asp Thr Gly Thr Pro His Trp Asn Asn Lys Glu
    450                 455                 460
Ala Leu Val Glu Phe Lys Asp Ala His Ala Lys Arg Gln Thr Val Val
465                 470                 475                 480
Val Leu Gly Ser Gln Glu Gly Ala Val His Thr Ala Leu Ala Gly Ala
                485                 490                 495
Leu Glu Ala Glu Met Asp Gly Ala Lys Gly Arg Leu Ser Ser Gly His
            500                 505                 510
Leu Lys Cys Arg Leu Lys Met Asp Lys Leu Arg Leu Lys Gly Val Ser
        515                 520                 525
Tyr Ser Leu Cys Thr Ala Ala Phe Thr Phe Thr Lys Ile Pro Ala Glu
    530                 535                 540
Thr Leu His Gly Thr Val Thr Val Glu Val Gln Tyr Ala Gly Thr Asp
545                 550                 555                 560
Gly Pro Cys Lys Val Pro Ala Gln Met Ala Val Asp Met Gln Thr Leu
                565                 570                 575
Thr Pro Val Gly Arg Leu Ile Thr Ala Asn Pro Val Ile Thr Glu Ser
            580                 585                 590
Thr Glu Asn Ser Lys Met Met Leu Glu Leu Asp Pro Pro Phe Gly Asp
        595                 600                 605
Ser Tyr Ile Val Ile Gly Val Gly Glu Lys Lys Ile Thr His His Trp
    610                 615                 620
His Arg Ser Gly Ser Thr Ile Gly Arg Val Phe Gln Lys Thr Lys Lys
625                 630                 635                 640
 
Gly Ile Glu Arg Leu Thr Val Ile Gly Glu His Ala Trp Asp Phe Gly
                645                 650                 655
Ser Ala Gly Gly Phe Leu Ser Ser Ile Gly Lys Ala Val His Thr Val
            660                 665                 670
Leu Gly Gly Ala Phe Asn Ser Ile Phe Gly Gly Val Gly Phe Leu Pro
        675                 680                 685
Lys Leu Leu Leu Gly Val Ala Leu Ala Trp Leu Gly Leu Asn Met Arg
    690                 695                 700
Asn Pro Thr Met Ser Met Ser Phe Leu Leu Ala Gly Gly Leu Val Leu
705                 710                 715                 720
Ala Met Thr Leu Gly Val Gly Ala
                725
SEQ. 11 - length 16

Met Val Gly Val Ala Leu Ala Leu Ala Leu Ala Thr Thr Ala Met Ala
1               5                   10                  15

## Claims

1. An amino acid sequence comprising SEQ ID NO: 9 or SEQ ID NO: 10, wherein each sequence comprises two natural sequences of Zika virus structural proteins in the form of prM structural protein and E structural protein or the cytoplasmic domain of E protein, and in addition comprises an optimized amino acid signal sequence optss shown as SEQ ID NO: 11, a GSG linker sequence linked to the P2A peptide, while the SEQ ID NO: 10 sequence comprises the helical and transmembrane domains of E protein of tick-borne encephalitis virus TBEV.

2. A recombinant flaviviral virus-like particle comprising SEQ ID NO: 9 or 10 for use in the prevention of infections caused by Zika virus of the Flavivirus genus, preferably as a vaccine component.

3. An antigen composition comprising at least one recombinant VLP with SEQ ID NO: 9 and/or SEQ ID NO: 10 for use in the prevention of infections caused by Zika virus of the Flavivirus genus.

## Patentansprüche

1. Aminosäuresequenz, umfassend SEQ ID-Nr. 9 oder SEQ ID-Nr. 10, wobei jede Sequenz zwei natürliche Sequenzen von Strukturproteinen des Zika-Virus in Form des prM-Strukturproteins und des E-Strukturproteins oder der zytoplasmatischen Domäne des E-Proteins umfasst und darüber hinaus eine optimierte Aminosäure-Signalsequenz (optss), dargestellt als SEQ ID-Nr. 11, eine GSG-Linkersequenz, die mit dem P2A-Peptid verknüpft ist, umfasst, wobei die Sequenz gemäß SEQ ID-Nr. 10 die helikalen und transmembranen Domänen des E-Proteins des Virus der durch Zecken übertragenen Enzephalitis (TBEV) umfasst.

2. Rekombinantes flavivirales virusähnliches Partikel, umfassend SEQ ID-Nr. 9 oder SEQ ID-Nr. 10, zur Verwendung bei der Prävention von Infektionen, die durch das Zika-Virus der Gattung *Flavivirus* verursacht werden, vorzugsweise als Impfstoffkomponente.

3. Eine Antigenzusammensetzung, umfassend mindestens ein rekombinantes virusähnliches Partikel (VLP) mit SEQ ID-Nr. 9 und/oder SEQ ID-Nr. 10, zur Verwendung bei der Prävention von Infektionen, die durch das Zika-Virus der Gattung *Flavivirus* verursacht werden.

## Revendications

1. Séquence d'acides aminés comprenant la SEQ ID NO : 9 ou la SEQ ID NO : 10, dans laquelle chaque séquence comprend deux séquences naturelles de proteins structurales du virus Zika sous la forme de la protéine structurale prM et de la protéine structurale E ou du domaine cytoplasmique de la protéine E, et comprend en outre une séquence signal d'acides aminés optimisée (optss), représentée par la SEQ ID NO : 11, une séquence de liaison GSG liée au peptide P2A, la séquence selon la SEQ ID NO : 10 comprenant les domaines hélicoïdaux et transmembranaires de la protéine E du virus de l'encéphalite à tiques (TBEV).

2. Particule pseudo-virale flavivirale recombinante, comprenant la SEQ ID NO : 9 ou la SEQ ID NO : 10, pour une utilisation dans la prévention des infections causées par le virus Zika du genre *Flavivirus,* de préférence en tant que composant vaccinal.

3. Composition antigène, comprenant au moins une particule pseudo-virale recombinante (VLP) avec la SEQ ID NO : 9 et/ou la SEQ ID NO : 10, pour une utilisation dans la prévention des infections causées par le virus Zika du genre *Flavivirus.*
